# EUROPEAN PATENT SPECIFICATION

(11) **EP 3 022 225 B1**
(45) Date of publication and mention of the grant of the patent: **29.09.2021**
(21) Application number: 14826647.1
(22) Date of filing: 21.07.2014
(51) Int. Cl.: C07K 16/18, C12Q 1/37, G01N 33/68, C12N 9/00

(54) **TRANSTHYRETIN AMYLOID-SELECTIVE AND POLYREACTIVE CATABODIES**
TRANSTHYRETINAMYLOID-SELEKTIVE UND POLYREAKTIVE KATALYTISCHE ANTIKÖRPER
ANTICORPS CATALYTIQUES POLYRÉACTIFS ET SÉLECTIFS D'AMYLOÏDE TRANSTHYRÉTINE

(30) Priority: 19.07.2013 US 201361856276 P
(43) Date of publication of application: 25.05.2016
(73) Proprietor: Board Of Regents Of the University Of Texas System, Austin, TX 78701 (US)
(72) Inventor: PAUL, Sudhir, Missouri City, TX 77459 (US); NISHIYAMA, Yasuhiro, Houston, TX 77030 (US); PLANQUE, Stephanie, Houston, TX 77054 (US)
(74) Representative: Schneiter, Sorin
(86) International application number: PCT/US2014/047409
(87) International publication number: WO 2015/010118

(56) References cited:
- WO-A1-2012/009709
- WO-A2-2008/034016
- WO-A2-2008/034016
- US-A1- 2006 094 063
- US-A1- 2009 155 254
- US-A1- 2014 056 904
- H. TAGUCHI ET AL: "Autoantibody-catalyzed Hydrolysis of Amyloid Peptide", JOURNAL OF BIOLOGICAL CHEMISTRY, vol. 283, no. 8, 22 February 2008 (2008-02-22), pages 4714-4722, XP055233461, US ISSN: 0021-9258, DOI: 10.1074/jbc.M707983200
- S. A. PLANQUE ET AL: "Physiological IgM Class Catalytic Antibodies Selective for Transthyretin Amyloid", JOURNAL OF BIOLOGICAL CHEMISTRY, vol. 289, no. 19, 9 May 2014 (2014-05-09), pages 13243-13258, XP055233464, US ISSN: 0021-9258, DOI: 10.1074/jbc.M114.557231
- PAUL A. RAMSLAND ET AL: "Crystal structure of a glycosylated Fab from an IgM cryoglobulin with properties of a natural proteolytic antibody", BIOCHEMICAL JOURNAL, vol. 395, no. 3, 1 May 2006 (2006-05-01), pages 473-481, XP055346630, GB ISSN: 0264-6021, DOI: 10.1042/BJ20051739
- SUDHIR PAUL ET AL: "Naturally Occurring Proteolytic Antibodies: SELECTIVE IMMUNOGLOBULIN M-CATALYZED HYDROLYSIS OF HIV gp120", JOURNAL OF BIOLOGICAL CHEMISTRY, AMERICAN SOCIETY FOR BIOCHEMISTRY AND MOLECULAR BIOLOGY, US, vol. 279, no. 38, 21 July 2004 (2004-07-21), pages 39611-39619, XP008153850, ISSN: 0021-9258, DOI: 10.1074/JBC.M406719200
- YUKIO ANDO ET AL: "Guideline of transthyretin-related hereditary amyloidosis for clinicians", ORPHANET JOURNAL OF RARE DISEASES, BIOMED CENTRAL LTD, LO, vol. 8, no. 1, 20 February 2013 (2013-02-20), page 31, XP021142605, ISSN: 1750-1172, DOI: 10.1186/1750-1172-8-31
- S. PLANQUE ET AL: "Ontogeny of Proteolytic Immunity: IgM SERINE PROTEASES", JOURNAL OF BIOLOGICAL CHEMISTRY, vol. 279, no. 14, 15 January 2004 (2004-01-15), pages 14024-14032, XP055346518, US ISSN: 0021-9258, DOI: 10.1074/jbc.M312152200
- TAGUCHI H ET AL: "Catalytic antibodies to amyloid beta peptide in defense against Alzheimer disease", AUTOIMMUNITY REVIEWS, ELSEVIER, AMSTERDAM, NL, vol. 7, no. 5, 1 May 2008 (2008-05-01), pages 391-397, XP022666650, ISSN: 1568-9972, DOI: 10.1016/J.AUTREV.2008.03.004 [retrieved on 2008-04-09]
- H. TAGUCHI ET AL: "Exceptional Amyloid Peptide Hydrolyzing Activity of Nonphysiological Immunoglobulin Variable Domain Scaffolds", JOURNAL OF BIOLOGICAL CHEMISTRY, vol. 283, no. 52, 26 December 2008 (2008-12-26), pages 36724-36733, XP055346615, US ISSN: 0021-9258, DOI: 10.1074/jbc.M806766200
- TAGUCHI ET AL.: 'Autoantibody-catalyzed Hydrolysis of Amyloid 13 Peptide' THE JOURNAL OF BIOLOGICAL CHEMISTRY vol. 283, no. 8, 17 December 2007, pages 4714 - 4722, XP055233461
- PLANQUE ET AL.: 'Physiological IgM class catalytic antibodies selective for transthyretin amyloid' J BIOL CHEM vol. 289, 19 March 2014, pages 13243 - 13258, XP055233464
- PAUL S ET AL: "SPECIFIC HIV GP120-CLEAVING ANTIBODIES INDUCED BY COVALENTLY REACTIVE ANALOG OF GP120", JOURNAL OF BIOLOGICAL CHEMISTRY, AMERICAN SOCIETY FOR BIOCHEMISTRY AND MOLECULAR BIOLOGY, vol. 278, no. 22, 28 March 2003 (2003-03-28), pages 20429-20435, XP008060874, ISSN: 0021-9258, DOI: 10.1074/JBC.M300870200
- GERSHONI JONATHAN M ET AL: "Epitope mapping - The first step in developing epitope-based vaccines", BIODRUGS, ADIS INTERNATIONAL LTD, NZ, vol. 21, no. 3, 1 January 2007 (2007-01-01), pages 145-156, XP009103541, ISSN: 1173-8804, DOI: 10.2165/00063030-200721030-00002

## Description

### FIELD OF THE INVENTION

The present invention relates generally to immunology. More specifically, the instant invention provides transthyretin amyloid-selective and polyreactive catabodies and methods of use thereof.

### BACKGROUND OF THE INVENTION

Several publications and patent documents are cited throughout the specification in order to describe the state of the art to which this invention pertains.

Amyloids are ancient threats. Jawed fish, the first extant organisms with an immune system orthologous to humans, produce TTR with sequence similar to human TTR. The human genome encodes no more than 100,000 non-antibody proteins, including all known enzymes and receptors with specificity for a broad range of ligands. The recombined antibody repertoire derived from the germline variable (V), D and J genes is larger by several orders of magnitude, suggesting the possibility of pre-existing specificity for defined targets in the innate antibody repertoire.

Protein self-aggregation forms amyloids with hydrogen-bonded β-sheets. This may fulfill useful functions, e.g., in bacterial curli and chaplains [6], but there is no known physiological function for age-associated accumulation of misfolded amyloids. Twenty seven misfolded proteins are associated with disease (e.g., TTR, Aβ, tau, α-synuclein, huntingtin, κ/λ light chains), and over 40 diseases are associated with misfolded proteins [1]. In the test-tube, particulate aggregates of amyloidogenic proteins are formed within minutes to days. Oligomeric aggregates of amyloidogenic proteins and subclinical tissue amyloid deposition can be detected decades prior to diagnosis of amyloidosis [7-9]. The soluble oligomers exert toxic effects via various receptors and membrane destabilization [10-12]. Particulate TTR amyloid deposits destroy tissue anatomic integrity. Aging is associated with accelerated protein misfolding. Factors thought to contribute to increased TTR amyloidosis include post-translational oxidation reaction [13] and binding to serum amyloid P component [14] or metals [15]. Localized wildtype TTR amyloidosis in certain anatomic areas is early sign of systemic ATTRwt, which usually appears in the sixth decade of life and becomes increasingly frequent thereafter [16]. Localized ATTRwt is frequent in carpal tunnel syndrome, skeletal dysfunction and lumbar stenosis [17, 18]. About 22-25% of humans over 80 years suffer from systemic ATTRwt, involving amyloidosis in the heart, lung, stomach, kidney, skin or other organs [19]. The rate of disease progression depends on the vulnerability of the affected area. Polyneuropathy and cardiomyopathy are frequent. Other than identifying tissue amyloid by invasive tissue biopsy, no unambiguous diagnosis method is available, and ATTRwt is thought to be an under-diagnosed disease. Several mutations in TTR can cause early onset hereditary ATTRm, including the Va1122Ile mutation affecting 3-4% of African Americans [20].

No approved drug is available to treat ATTRwt or ATTRm. The standard of clinical care is replacing the failed organ by transplantation (or in ATTRm, transplantation of the liver, the major TTR synthesis site). Tafamidis, a small molecule that binds the T4-binding site of phyTTR [21], may reduce neuropathy in familial amyloidosis [22] but was not approved in the United States because of questionable efficacy. Anti-sense oligonucleotide and short-interfering RNA approaches that inhibit TTR synthesis were reported [23, 24], but these drugs may interfere with the physiological TTR functions. Inducing dissociation of bound serum amyloid P component (SAP) was suggested as a means to clear TTR amyloid [14]. Catechin, a green tea anti-oxidant, and a doxycycline/tauroursodeoxycholic acid combination were also suggested as treatments [25, 26]. However, it is clear that new and improved methods of treating TTR amyloidosis are needed.

### SUMMARY OF THE INVENTION

The invention describes novel IgM class antibodies in healthy humans with catalytic activity (catabodies) with a unique specificity for the misfolded form of transthyretin (misTTR) responsible for forming misTTR amyloid deposits. The catabodies dissolved particulate misTTR. Development of specificity in classical IgG antibodies requires immunization with the target antigens. IgGs did not hydrolyze misTTR. The specificity of the misTTR-directed IgM catabodies is an innate property that has evolved by Darwinian evolution. The physiologically folded form of transthyretin was not hydrolyzed by the IgM catabodies. By virtue of the peptide bond hydrolysis and product release reactions, detectable immune complexes were not formed. Also disclosed are human monoclonal misTTR-directed catabodies cloned from B cells; electrophilic misTTR analogs used for preparing B cell lines producing the misTTR-directed catabodies and polyreactive catabodies; and methods to prepare the catabodies and the electrophilic analogs. The sera of aged humans with an established diagnosis of TTR amyloidosis displayed reduced misTTR-directed catabody activity accompanied by increased polyreactive catabody activity, suggesting the potential of the catabody tests for diagnosis and prognosis of developing amyloid disease. Specific catabodies may be conceived as components of a natural defense system that remove pathogenic self-antigens. Supplementing the endogenous catabodies with a misTTR-specific monoclonal catabody holds therapeutic potential.

In accordance with an aspect, the instant invention provides the isolated catalytic antibody or fragment thereof of claim 1. The catalytic antibodies of the instant invention catalyze the hydrolysis of misfolded transthyretin, particularly without hydrolysis of the properly folded transthyretin. In a particular embodiment, the catalytic antibody is an IgM or a fragment thereof. In a particular embodiment, the catalytic antibody is innate. The misfolded transthyretin comprises soluble oligomers or particulate amyloid with β-structure. The misfolded transthyretin may be mammalian, particularly human.

In accordance with another aspect, the instant invention provides an *in vitro* method for detecting misfolded transthyretin in a test sample in accordance with claim 5. In a particular embodiment, the method comprises contacting the test sample with a catalytic antibody or fragment thereof of the instant invention and monitoring the hydrolysis of misfolded transthyretin contained in the test sample by detecting the presence of smaller hydrolysis products.

In accordance with another aspect, the instant invention provides the *in vitro* method for diagnosing transthyretin amyloidosis in accordance with claim 6. In a particular embodiment, the misfolded transthyretin is detected using the catalytic antibodies or fragments thereof of the instant invention.

In accordance with another aspect, the instant invention provides the electrophilic analog of misfolded transthyretin of claim 7. The method of claim 8 for isolating catalytic antibodies that hydrolyze misfolded transthyretin is also provided.

The invention further provides the electrophilic analog of the invention as a vaccine in the treatment or prevention of transthyretin amyloidosis. The invention also provides the electrophilic analog of the invention for use in the production of monoclonal catalytic antibodies.

### BRIEF DESCRIPTIONS OF THE DRAWING

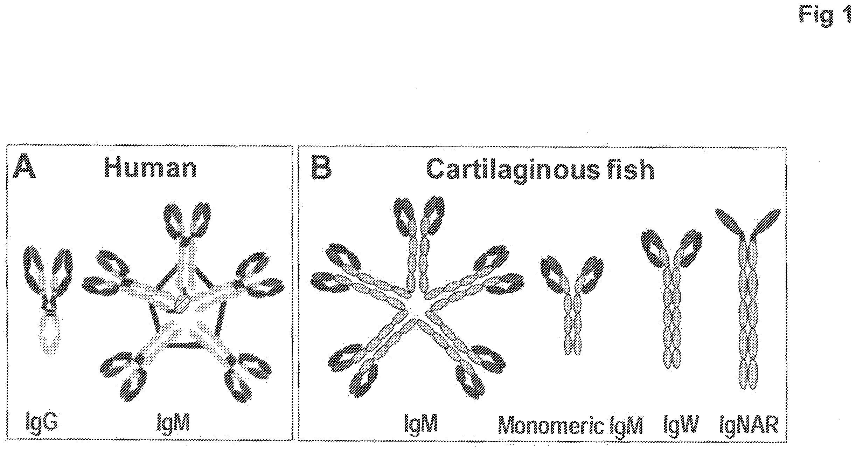

Figures 1A and 1B show the structure and phylogeny of immunoglobulins [54]. Figure 1A shows human IgG and IgM domain organization. Figure 1B shows cartilaginous fish domain organization. Heavy and light chain C-domains and V-domains are shown. The fish do not produce IgGs. IgM exists both as monomers and pentamers. IgW refers to an isoform containing a long 7 domain (shown) and a short 3 domain heavy chain form. IgNAR (NAR, novel antigen receptor) contains unpaired V-domains.

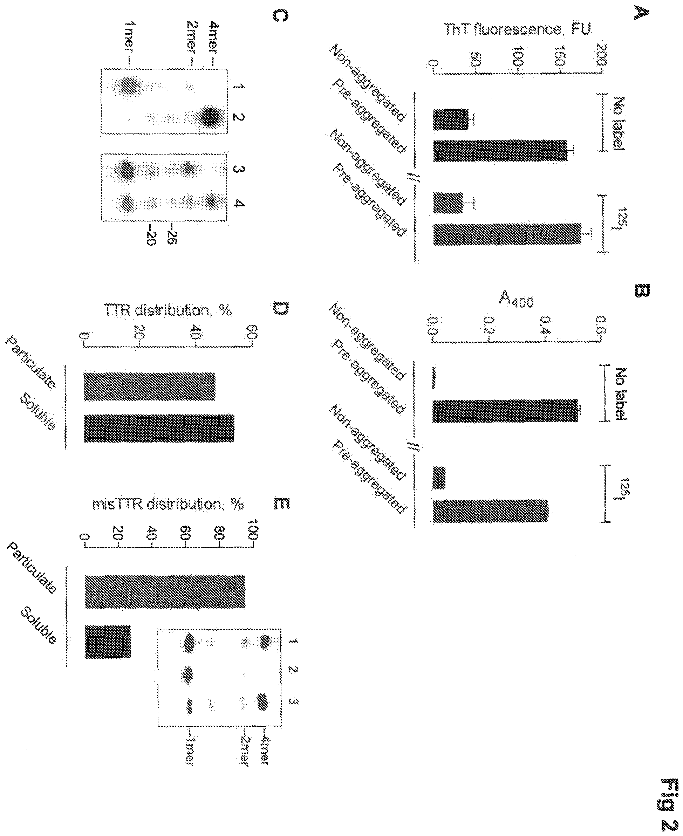

Figures 2A-2E show pre-aggregated and non-aggregated TTR properties. Figure 2A shows ThT binding. ThT fluorescence values for non-radiolabeled TTR ("no label") and ¹²⁵I-TTR ("¹²⁵I") were small compared to TTR aggregated over 5 days. TTR, 7.1 µM; ThT, 15 µM. Figure 2B shows turbidity. The turbidity of non-radiolabeled TTR ("no label") and ¹²⁵I-TTR ("¹²⁵I") was marginal compared to the TTR aggregated over 5 days. Figure 2C shows SDS-electrophoresis. Left box, non-aggregated ¹²⁵I-TTR without (lane 2) or with boiling (lane 1). Right box, pre-aggregated ¹²⁵I-TTR without (lane 4) or with boiling (lane 3). Bands labeled 4mer, 2mer and 1mer are the tetramer (54 kDa), dimer (27 kDa) and monomer (14 kDa), respectively. Minor 20 and 26 kDa bands are impurities from the source TTR (<0.5%). The 4mer phyTTR species is dominant in non-boiled, non-aggregated TTR, whereas the 1mer derived from soluble and insoluble misTTR is present in large amounts in the non-boiled, pre-aggregated TTR. misTTR content of the pre-aggregated and non-aggregated ¹²⁵I-TTR preparation, respectively, 70% and <0.5%. Figure 2D provides graphs of TTR distribution in particulate and soluble fractions. Nearly equivalent radioactivity amounts from pre-aggregated ¹²⁵I-TTR were recovered in the pellet (particulate fraction) and supernatant (soluble fraction) obtained by centrifugation. Figure 2E shows misTTR distribution in particulate and soluble TTR fractions. The non-boiled particulate and soluble fractions prepared from pre-aggregated ¹²⁵I-TTR contained the radioactive 1mer band derived from misTTR as the majority and minority species, respectively. Plotted values computed as 100 x [1mer band intensity/[sum of 1mer and 4mer band intensities]. Inset: Lane 1 shows pre-aggregated ¹²⁵I-TTR without separation into particulate and soluble fractions (radioactivity in 1mer and 4mer bands, respectively, 43% and 57%). The 1mer band is dominant in particulate ¹²⁵I-TTR (95% of total radioactivity; lane 2) and is also present in smaller amounts in soluble ¹²⁵I-TTR (27% of total radioactivity; lane 3).

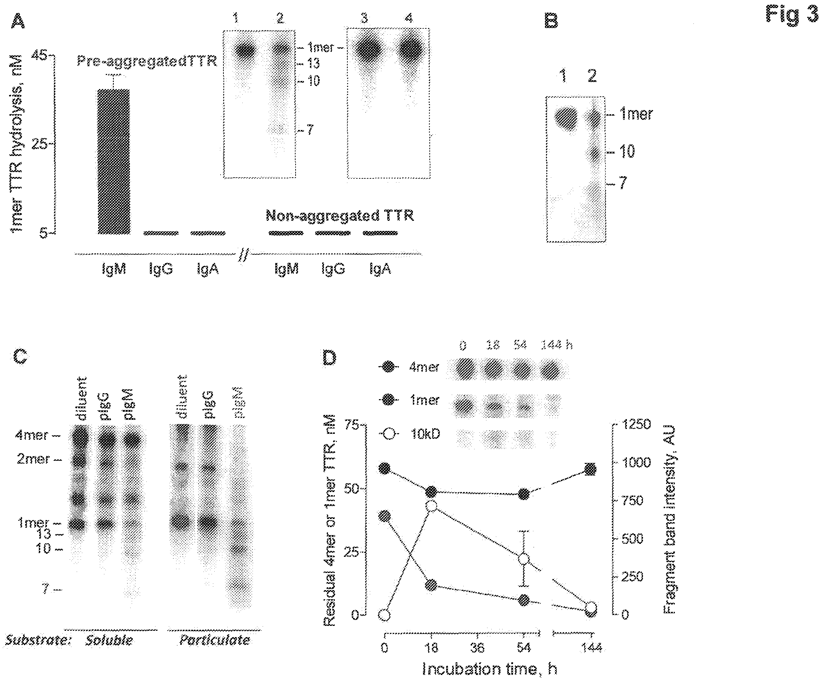

Figures3A-3D show selective misTTR hydrolysis by polyclonal IgM. Figure 3A shows IgM class-restricted hydrolytic activity. Boiled reaction mixtures of pre-aggregated or non-aggregated ¹²⁵I-TTR (100 nM; misTTR content, 40%) treated for 18 h with diluent or IgM, IgG and IgA purified from human serum (130 µg/mL, pooled from sera of 12 healthy humans) were electrophoresed. Hydrolysis was determined from depletion of the 1mer band intensity after antibody treatment relative to the band intensity after diluent treatment. Inset: electrophoresis lanes showing depletion of the 1mer band in pre-aggregated ¹²⁵I-TTR treated with IgM (lane 2) compared to diluent (lane 1). Products are visible as 13, 10, and 7 kDa bands. No 1mer band depletion or products were evident in the pIgM-treated, non-aggregated ¹²⁵I-TTR (lane 4) compared to the diluent-treated substrate (lane 3). Figure 3B shows the hydrolysis of non-radiolabeled, misTTR by polyclonal IgM. Silver-stained electrophoresis gel of pre-aggregated TTR (4.5 µM) treated for 52 h with diluent (lane 1) or pooled pIgM (lane 2; 570 µg/mL). From densitometry, 85% of the 1mer band derived from misTTR was depleted by pIgM treatment. The 10 and 7 kDa product fragments are observed. The 13 kDa product is not visible because of poor silver stain ability. Figure 3C shows the hydrolysis of soluble and particulate misTTR species by pIgM. The soluble and particulate fractions of pre-aggregated ¹²⁵I-TTR (misTTR content, respectively, 37% and >95%) were treated for 18 h with diluent or polyclonal IgM (pIgM) and IgG (pIgG) purified from the same human serum pool (130 µg/mL). Non-boiled reaction mixtures were analyzed. Figure 3D shows misTTR selectivity. The 1mer band (14 kDa) derived from misTTR but not the 4mer phyTTR band (54 kDa) of pre-aggregated ¹²⁵I-TTR (100 nM; misTTR content, 40%) was depleted as a function of time by treatment with pooled pIgM (130 µg/mL). Inset: 4mer, 1mer and 10 kDa band cut-outs from electrophoresis gels. Non-boiled reaction mixtures.

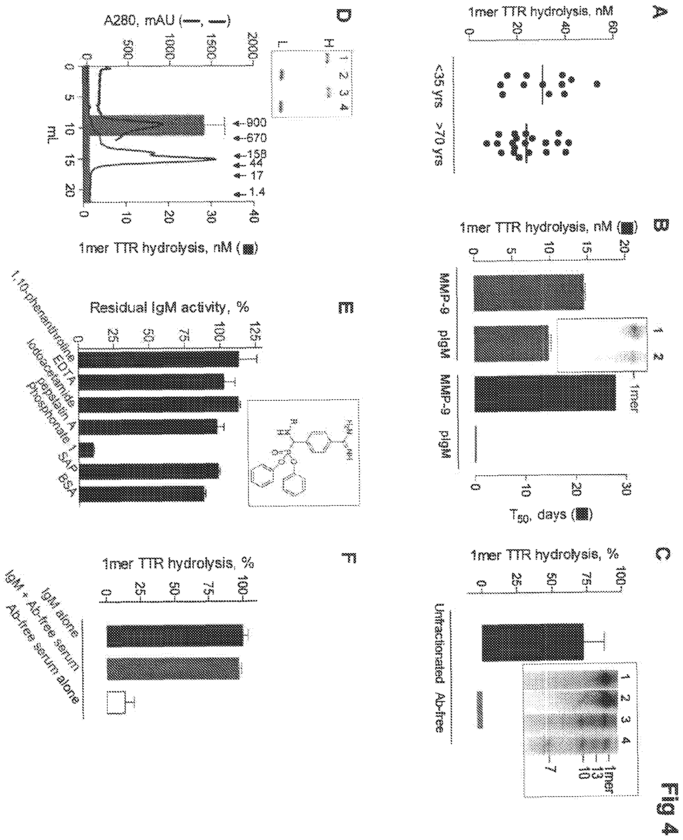

Figures 4A-4F show the characterization of polyclonal IgM hydrolytic activity. Figure 4A shows the comparison of hydrolytic activity of individual pIgM preparations (90 µg/mL) from non-aged (<35 years, n=12) and aged humans (>70 years, n=20) determined by electrophoresis. Pre-aggregated ¹²⁵I-TTR substrate (160 nM; misTTR content, 60%; 18 h incubation). Non-boiled reaction mixtures. Figure 4B shows the rates of pre-aggregated ¹²⁵I-TTR hydrolysis by MMP-9 and pIgM (left axis). MMP9 and pIgM concentrations, 133 nM. Other conditions as in Figure 4A. T50 (right axis) is the time required to hydrolyze 50% of 1 nM misTTR at the MMP9 or pIgM concentrations in blood (respectively, 3.2 nM and 2.2 µM) computed from the equation S=S₀(1-e^{-[Eo]kt} ), where So is substrate concentration at time 0, E0 is the enzyme concentration at time 0, k is the second order rate constant derived from the equilibrium binding and catalytic constants, and t is reaction time. Inset: SDS-gel showing reduced 1mer misTTR band following MMP9 treatment (lane 2) compared to diluent treatment (lane 1). Figure 4C shows pIgM immunoadsorption. misTTR hydrolysis by unfractionated human serum and antibody (Ab)-free serum was measured as in Figure 4A. The protein content of unfractionated serum (diluted 1:100) and the antibody-free serum was adjusted to be identical (1.14 mg/mL). IgM concentrations in hydrolysis assays using unfractionated serum and antibody-free serum were 20 and <0.01 µg/mL, respectively. Data are expressed as % depletion of the 1mer misTTR band compared to the control substrate reaction mixture treated with diluent (100% value = 22.5±5.6 nM). Inset: electrophoresis showed identical product fragments (13, 10 and 7 kDa bands) generated by digesting pre-aggregated ¹²⁵I-TTR with unfractionated serum (lane 3, 1:100 dilution) and purified IgM (lane 4, 120 µg/mL). Lane 1, substrate treated with diluent. Lane 2, substrate treated with antibody-free serum. Figure 4D shows distribution of hydrolytic activity of unfractionated serum. Only the IgM-containing 900 kDa fraction (retention volume 8-11 mL) obtained by FPLC gel filtration of human serum displayed detectable misTTR hydrolytic activity assayed as in Figure 4A. IgM concentration in the assay, 20 µg/ml (equivalent to IgM concentration in 1% unfractionated serum). Elution of reference purified IgM is coincident with elution of the serum hydrolytic activity. Non-IgM serum fractions were tested as 2 pools at concentration corresponding to 1% unfractionated serum equivalents (retention volume 0-8.0 mL and 11.0-22.0 mL; concentrated using a 3 kDa ultrafilter). Inset: reducing SDS-gels of 900 kDa serum IgM fraction showing anti-µ antibody stained 70 kDa heavy chain band (lane 1) and anti-λ/κ antibody stained 25 kDa light chain band (lane 2). Lanes 3 and 4, respectively, are similarly-stained heavy and light chain bands of the reference IgM, respectively. H, heavy chain; L, light chain. Figure 4E shows protease inhibitor effects. misTTR hydrolysis was measured using IgM pretreated (1 h) with diluent or the indicated inhibitors of metalloproteases, cysteine proteases, acid proteases and serine proteases. The structure of E-hapten 1 is shown. SAP is an amyloid binding protein. Residual hydrolytic activity of inhibitor-treated IgMs was computed as % of IgM activity after treatment with diluent (100% value = 24±4 nM TTR). Hydrolysis was measured using boiled reaction mixtures of 100 nM pre-aggregated ¹²⁵I-TTR incubated with 60 µg IgM/mL for 18 h. Figure 4F shows unimpeded hydrolysis of pre-aggregated ¹²⁵I-TTR by pIgM in the presence of antibody (Ab)-free serum. Plotted are 1mer misTTR hydrolytic activities expressed as % of the activity of purified (130 µg/mL) alone (90±3 nM). Reactions conditions as in Figure 4A.

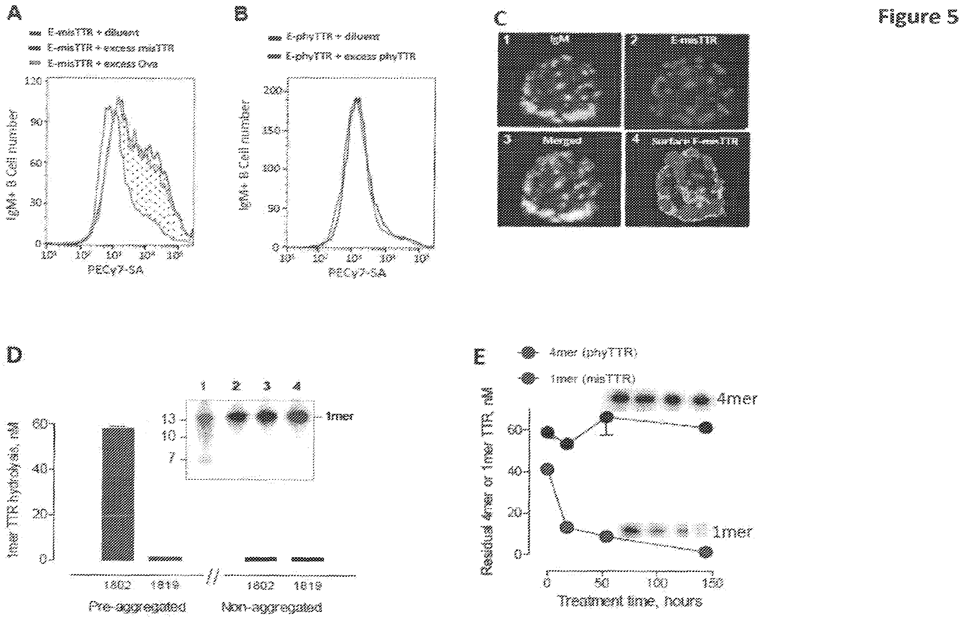

Figures 5A-5E show selective misTTR recognition by B cells and monoclonal IgMs. Figure 5A shows selective E-misTTR binding by human IgM+ B cells. Profile of PECy7-SA stained IgM+ B cells treated with E-misTTR in diluent or excess non-biotinylated misTTR. Dotted area represents IgM+ B cells with saturable E-misTTR binding activity (45% of total IgM+ B cells). E-misTTR binding was not affected in the presence of excess ovalbumin (Ova). Figure 5B shows near-equivalent binding of the cells to E-phyTTR was evident in diluent and presence of excess phyTTR, suggesting the absence of saturable E-phyTTR reactivity. Figure 5C shows E-misTTR binding to IgM+ B cells. Deconvoluted images showing an IgM+ B cell stained with FITC-conjugated antibody to IgM (image 1), the cell stained PECy7-SA to visualize E-misTTR binding (image 2), the merged rendition (image 3) and the reconstituted 3D model suggesting E-misTTR binding to cell surface BCRs (image 4). Counterstain, 4',6-diamidino-2-phenylindole. 60X. Figure 5D shows misTTR hydrolysis by monoclonal IgM 1802. The reaction mixtures of pre-aggregated or non-aggregated ¹²⁵I-TTR (100 nM) treated with diluent or hydrolytic mIgM 1802 (18 h, 60 µg/mL) were boiled, thereby dissociating both phyTTR and misTTR into 1mers (14 kDa). IgM 1819 is one of 4 IgMs devoid of misTTR hydrolytic activity. Inset, Electrophoresis gel showing pre-aggregated ¹²⁵I-TTR treated with IgM 1802 (lane 1) or mIgM 1819 (lane 2), and non-aggregated ¹²⁵I-TTR treated with mIgM 1802 (lane 3) or mIgM 1819 (lane 4). Product profiles were similar to the polyclonal IgM digests. Figure 5E shows time-dependent misTTR hydrolysis. mIgM 1802 treatment (120 µg/mL) depleted the 1mer band derived from misTTR but not the 4mer phyTTR band visualized by electrophoresis (assay conditions as in Fig 4A. Insets: Electrophoresis gel cut-outs of the 4mer phyTTR band and 1mer misTTR band at the 4 time points tested (left to right: 0, 18, 54, and 154 h).

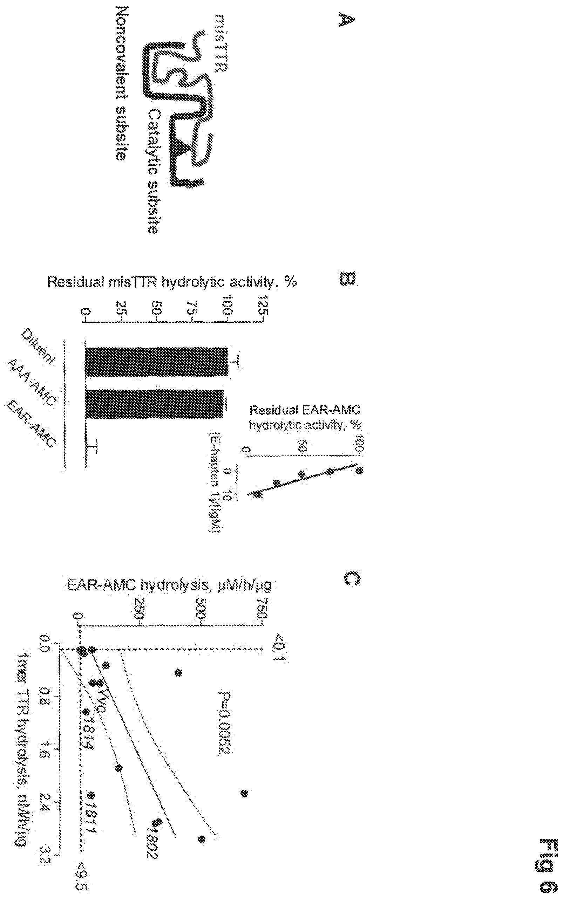

Figures 6A-6C show the relationship between EAR-AMC and misTTR hydrolysis by monoclonal IgMs. Figure 6A shows split-site catabody model. Catabody selectivity derives from initial epitope recognition by the noncovalently binding subsite followed by peptide bond hydrolysis at a spatially proximate catalytic subsite. Small peptide substrates are hydrolyzed at the catalytic subsite without dependence on the noncovalent binding subsite. Figure 6B shows misTTR and EAR-AMC hydrolysis at a shared catalytic subsite. Hydrolysis of pre-aggregated ¹²⁵I-TTR (100 nM; misTTR content, 40%) by mIgM Yvo (300 µg/mL) was measured in the presence of the alternate substrates EAR-AMC or AAA-AMC (1 mM; incubation for 18 h). IgM hydrolytic activity is expressed as % of activity in diluent instead of alternate substrate (45±3 nM).Inset, Data from [55] showing stoichiometric inhibition of mIgM Yvo-catalyzed EAR-AMChydrolysis at varying E-hapten 1/IgM molar ratios. X-intercept is the number of catalytic sites/mIgM molecule. Figure 6C shows correlated EAR-AMC and misTTR hydrolysis by mIgMs. EAR-AMChydrolysis by the panel of 16 mIgMs was measured fluorimetrically [37]. misTTR hydrolysis was measured as in Fig. 5 using boiled reaction mixtures. Solid and dotted lines show the least-square regression fit (Pearson r² 0.44, P<0.005) and 95% confidence band, respectively.

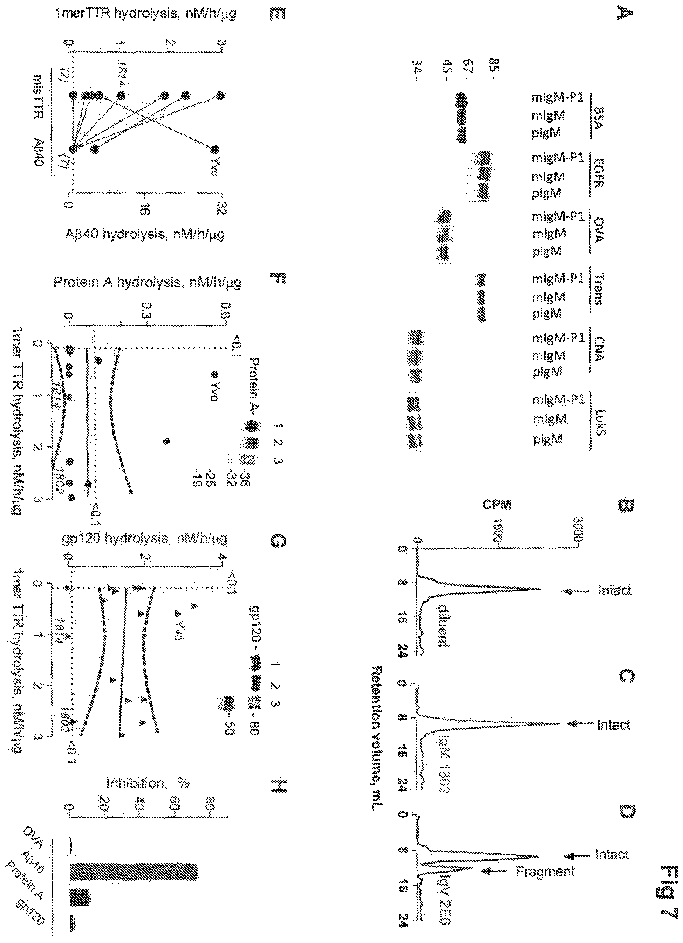

Figures 7A-7H show monoreactive and oligoreactive misTTR hydrolyzing monoclonal IgMs. Figure 7A shows no hydrolysis of non-amyloid and non-superantigen proteins. SDS-electrophoresis gels containing reaction mixtures of the following biotinylated proteins treated for 22 h with pIgM (pooled from 12 humans) or mIgM 1802 (130 µg/ml): extracellular domain of epidermal growth factor receptor (exEGFR), bovine serum albumin (BSA), ovalbumin (OVA), transferrin (Trans) and two Staphylococcus aureus virulence factors, LukS and collagen adhesion protein CNA (CNA). mlgM-P1 represents the control reaction mixture containing IgM 1802 inhibited by E-hapten 1. Figures 7B-7D show no fibrillar Aβ hydrolysis by mIgM 1802. FPLC gel filtration profiles of formic acid-solubilized reaction mixtures containing fibrillar ¹²⁵I-Aβ42 (30,000 cpm) treated for 16 h with diluent (Fig. 7B), monoclonal mIgM 1802 (120 µg/mL) (Fig. 7C) or the Aβ-hydrolyzing immunoglobulin V domain fragment (IgV 2E6, 10 µg/mL) (Fig. 7D). No depletion of intact ¹²⁵I-Aβ42 (computed mass 4,630 Da) or product appearance was evident by mIgM treatment, whereas IgV 2E6 generated a radioactive fragment with mass 1654 Da corresponding to hydrolysis at the Hisl4-Gln15 bond [33]. Figure 7E shows discordant misTTR and Aβ hydrolytic activities of mIgMs (n=9). Aβ40 hydrolysis data from [55]. Connecting lines identify individual mIgMs. In parentheses are the number of mIgMs without detectable activity.misTTR-monoreactive mIgM 1814 and oligoreactive mIgM Yvo are identified. Figure 7F shows discordant misTTR and Protein A hydrolytic activities of mIgMs (n=16). Hydrolysis of biotinylated Protein A (72 nM) following IgM treatment (45 µg/mL, 72 h) was determined electrophoretically. Solid and dotted lines are the least-square regression fit and 95% confidence bands, respectively (P>0.05, r²<0.001; 2-tailed Pearson analysis). misTTR-monoreactive mIgMs 1802 and 1814 and oligoreactive mIgM Yvo are identified.Inset, SDS-electrophoresis lanes of Protein A treated with diluent (lane 1), non-hydrolytic mIgM 1801 (lane 2) and hydrolytic mIgM Yvo showing depletion of intact Protein A and appearance of smaller-mass products (lane 3). Figure 7G shows hydrolysis of biotinylated gp120 (100 nM) following IgM treatment (45 µg/mL, 18 h) was determined electrophoretically. Solid and dotted lines are the least-square regression fit and 95% confidence bands, respectively (P>0.05, Pearson r2 0.007). misTTR-monoreactive mIgMs 1802 and 1814 and oligoreactive mIgM Yvo are identified.Inset, SDS-electrophoresis of gp120 treated with diluent (lane 1), non-hydrolytic mIgM 1801 (lane 2) and hydrolytic mIgM Yvo showing depletion of intact gp120 and appearance of smaller-mass product bands (lane 3). Figure 7H shows inhibition of mIgM Yvo catalyzed misTTR hydrolysis by alternate substrates. Only Aβ inhibited the misTTR hydrolytic activity. Like the irrelevant protein ovalbumin (OVA), the superantigens Protein A and gp120 were non-inhibitory. Alternate substrates, 1 µM; mIgM Yvo, 130 µg/mL; other reaction conditions as in Fig 5A.

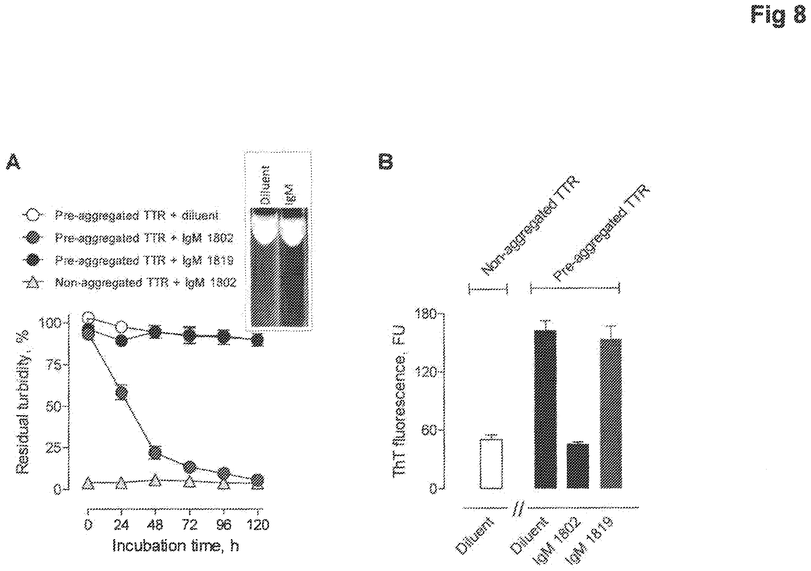

Figures 8A-8B show the dissolution of pre-aggregated TTR by mIgM 1802. Figure 8A shows reduced turbidity. Pre-aggregated TTR (7.1 µM) was incubated in the presence of hydrolytic mIgM 1802 (120 µg/mL, 0.67 µM), an equivalent non-hydrolytic IgM 1819 concentration or diluent. mIgM 1802 treatment reduced the turbidity to basal value observed for control non-aggregated TTR treated with mIgM 1802. Inset, Photograph showing turbid misTTR suspension (white precipitates against black background) following incubation in diluent and misTTR dissolution after treatment with the hydrolytic IgM. Figure 8B shows reduced ThT fluorescence. Shown are ThT fluorescence values after treating pre-aggregated TTR with hydrolytic mIgM 1802, non-hydrolytic mIgM or diluent for 120 h as in panel A. mIgM 1802 treatment reduced the ThT fluorescence value to the basal value observed for non-aggregated TTR treated with diluent. Data are corrected for ThT fluorescence of IgMs alone without TTR substrate (84±5 FU).

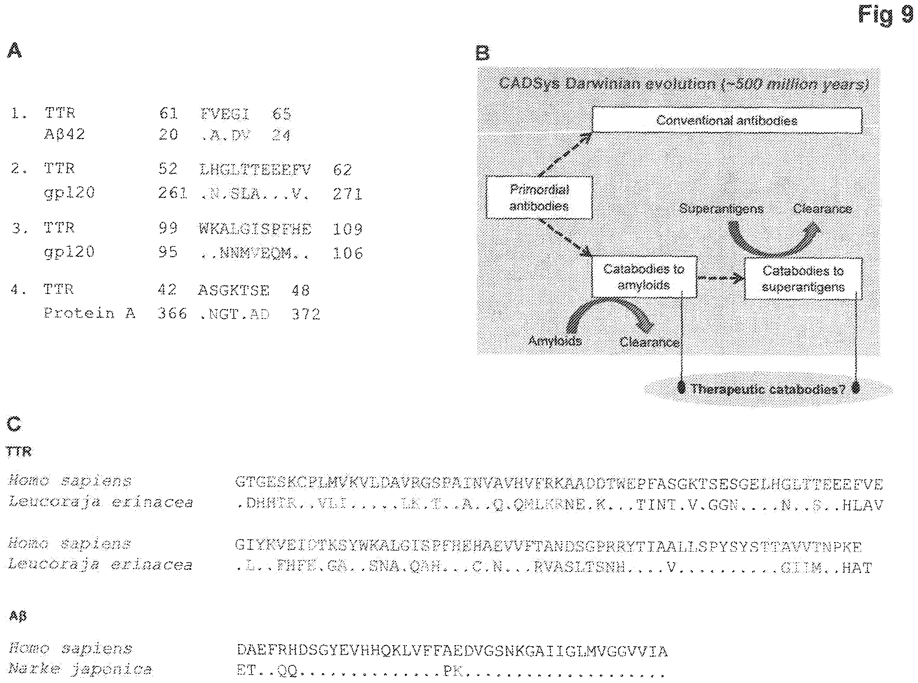

Figures 9A-9C show the catabody defense system (CADSys). Figure 9A shows regions of TTR with best sequence similarity to Aβ, gp120 and Protein A. 1, TTR and Aβ42 (e=9.4). 2 and 3, TTR and gp120 (e=2.6 and 6.4, respectively). 4, TTR and Protein A (e=23). Dots: identities. Shaded: conservative substitutions. Alignments were obtained with Blast (blast.ncbi.nlm.nih.gov/) using BLOSUM62 matrix and expect (e) threshold of 1000. The sequence similarities are weak (large e values, P>0.05 for all alignments). The indicated Protein A region, but not the Aβ42 and gp120 regions, has a β-turn forming propensity (tools.immuneepitope.org/tools/ bcell/iedb_input). The TTR region aligned with Protein A also tends to form a β-turn. Substrate regions with greatest β-turn forming propensity are (score> 1.0): TTR, 95-101 and 109-115; Aβ, 5-11 and 23-29; gp120, 146-147e and 362-368 (HXB2 numbering); Protein A, 259-265 and 299-305. Accession numbers: human TTR, AAA73473; human Aβ42, NP000475; HIV gp120MN, AF075721; S. aureusProtein A, 88193823. Figure 9B shows the CADSys concept is based on discovery of innate antibodies that clear pathogenic amyloids and microbial superantigens. As amyloid removal is predicted to confer a survival advantage to the organism, emergence of germline anti-amyloid catabodies in immune evolution is proposed. Superantigen epitopes leave microbes susceptible to innate catabodies and reversibly-binding antibodies. However, superantigens help microbes evade acquired immunity, and microbial superantigen-host immune system interactions likely reflect competing factors favoring survival of microbes and the host. Figure 9C shows amyloid protein conservation in humans and jawed fish of the Batoidea superorder (Leucorajaerinacea,Narke japonica). Top, TTR and bottom, Aβ42. Dots: amino acid identities. Shaded: conservative substitutions. The full-length human TTR and Aβ42 sequences are shown. Accession numbers: Leucorajaerinacea TTR, CV221819; Narke japonica amyloid precursor protein 699 residues 601-642, BAA24230.1. Jawed fish are the first extant organisms with human antibody-like molecules.

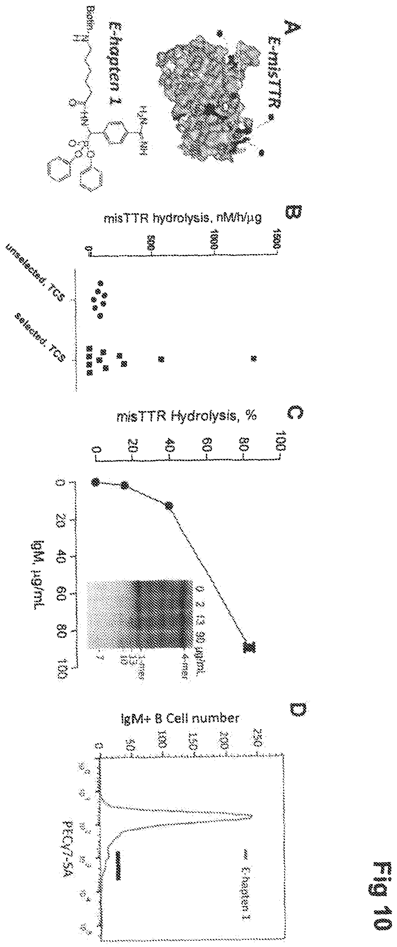

Figures 10A -10D show isolation of monoclonal catabodies from healthy humans as purified IgMs or tissue culture supernatant (TCS) . Figures 10A show TTR (E-misTTR) used for B cell selection contains electrophilic phosphonates and biotin substituents linked to Lys residues. The E-hapten-1 is the small molecule biotinylated electrophilic phosphonate. Figure 10B show four cell lines prepared by EBV transformation of B cells selected by E-misTTR secreted monoclonal IgMs that hydrolyzed misTTR at rates superior to unselected B cells as determined by the electrophoresis cleavage assay. Unfractionated culture supernatants of equivalently cultured transformed monoclonal cell lines with verified IgM secreting activity were tested. Supernatants from control B cell lines devoid of IgM secretory activity consistently failed to hydrolyze misTTR. Figure 10C shows dose-dependent hydrolysis of misTTR by the E-misTTR-selected IgM clone 10E10. Reaction mixtures of pre-aggregated ¹²⁵I-TTR (100 nM misTTR) treated for 18 h with diluent or increasing concentration of IgM purified from tissue culture supernatant were subjected to SDS-electrophoresis without boiling as in Example 1. Inset, Electrophoresis lanes showing depletion of the 1mer band derived from misTTR in pre-aggregated ¹²⁵I-TTR upon treatment with IgM 10E10. Products are visible as 13, 10, and 7 kDa bands. The band intensity of the 4mer corresponding to phyTTR is unchanged. Figures 10D show the horizontal bar identifies the E-hapten 1 bound IgM+ B cell subset analyzed by flow cytometry(PECy7-SAstained cells; methods as in Example 1).

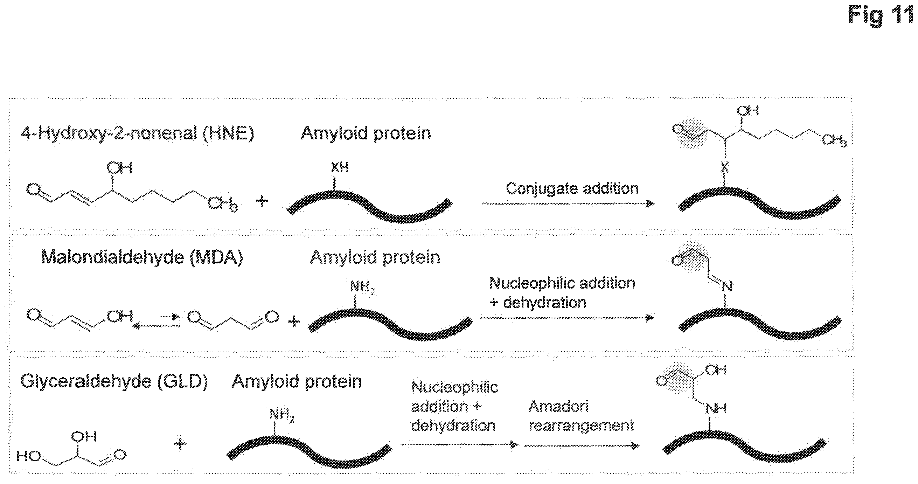

Figure 11 shows the representative protein carbonylation reactions. HNE, MDA and GLD are advanced lipid peroxidation/glycation end-products reactive with amino acid side chains (-XH = His-Nim, Lys-Nε, or Cys-S; -NH₂ = Lys-Nε) via Michael addition (top), Schiff's base formation (middle) or Amadori reaction (bottom). The protein adducts contain an electrophilic carbonyl (circles).

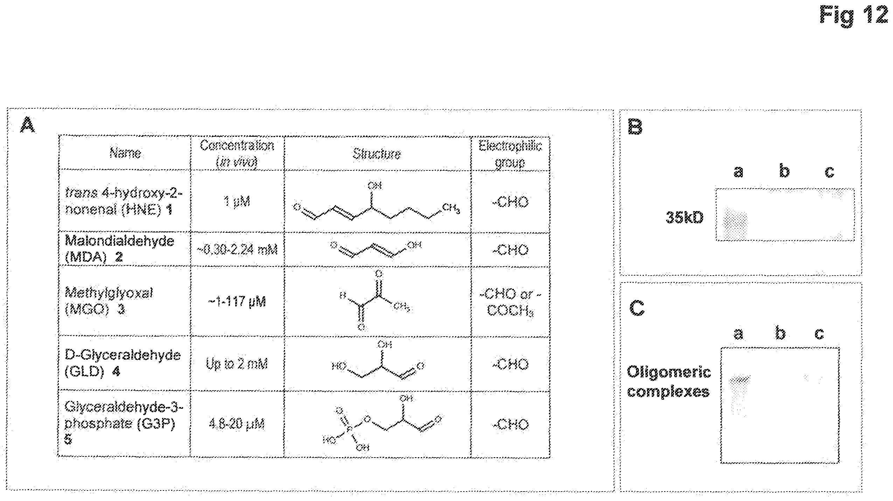

Figures 12A-12C show the structure of advanced lipid peroxidation and glycation end products and reactivity with catalytic IgV 2E6. Figure 12A shows advanced lipid peroxidation and glycation endproducts used for preparing naturally-occurringelectrophilic amyloid probes. Figures 12B and 12C show irreversible NE-Aβ42 binding by IgV 2E6.NE-Aβ42 was prepared by treatment with malondialdehyde. Figure 12B shows monomer NE-Aβ42complexed to the IgV (35 kDa)revealed by anti-Aβ staining following treatment with IgV 2E6 (lane a), noncatalytic IgV MMF6 (laneb) or diluent (lane c). IgV, 20 µg/mL; NE-Aβ42, 2.5 µM. Figure 12C shows oligomeric complexes of NE-Aβ42 with the IgV close to the loading position in the gel identified by staining of the IgV c-myc tag (lane a). Lane b shows the reaction mixture of IgV 2E6 and with Aβ42that had not been derivitized with malondialdehyde. Lane c shows the reaction mixture of noncatalytic IgV MMF6with NE-Aβ42.

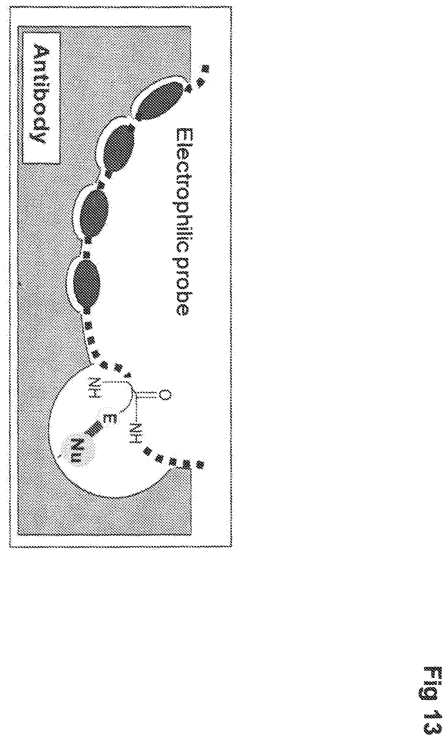

Figure 13 shows a model of covalent binding between antibody and an electrophilic Aβ analog. Noncovalent epitope binding (blue beaded structure) to the paratope occurs in coordination with nucleophilic attack by another antibodysubsite (Nu). The electrophile (E) is a surrogate for the peptide bond carbonyl and forms a non-hydrolyzable covalent bond with Nu.

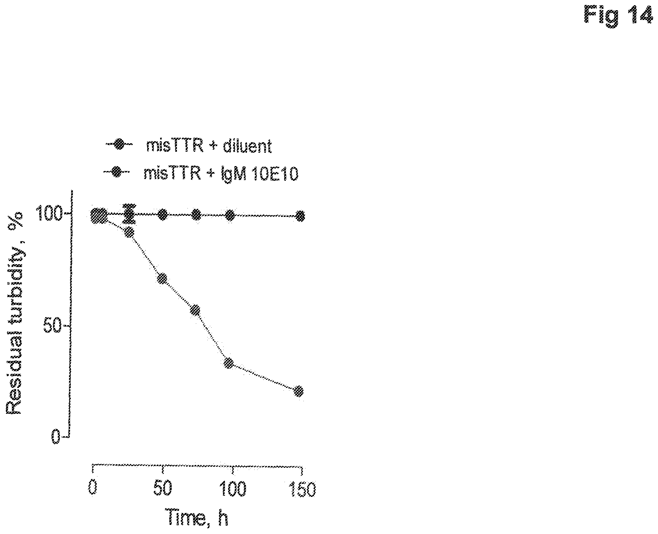

Figure 14 shows dissolution of TTR amyloid by monoclonal IgM 10E10. Pre-aggregated TTR (22 µg/mL) was incubated with diluent or catalytic IgM 10E10 (100 µg/mL), and turbidity (400 nm) was measured as in Example 1.

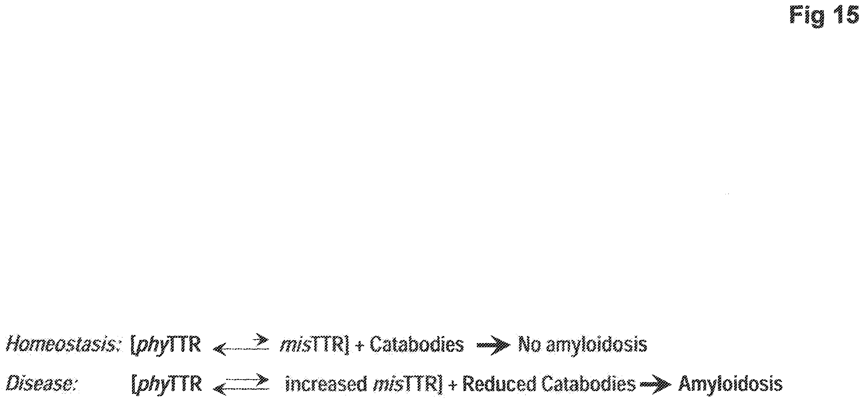

Figure 15 shows homeostatic control of TTR amyloidosis.

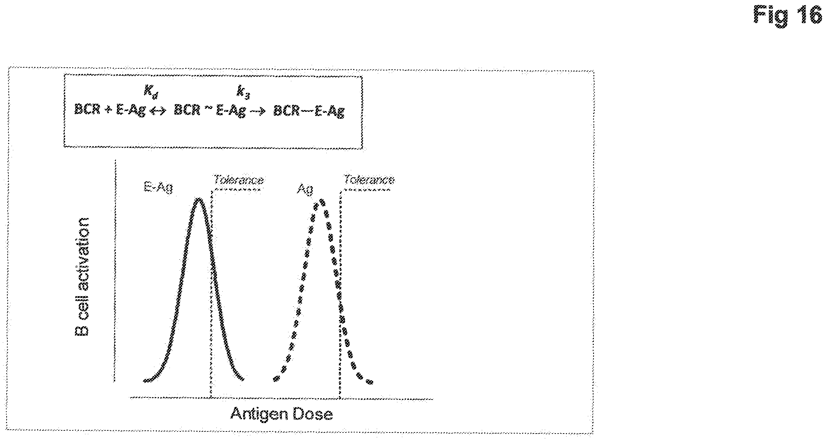

Figure 16 shows B cell tolerance induction by electrophilic antigen (E-Ag). E-Ag (left) and antigen (Ag, right) dose response curves. As the covalently reactive E-Ag saturates BCRs more efficiently, the dose response curve shifts to the left. Inset, E-Ag reaction scheme.-, noncovalent binding; -, covalent binding.

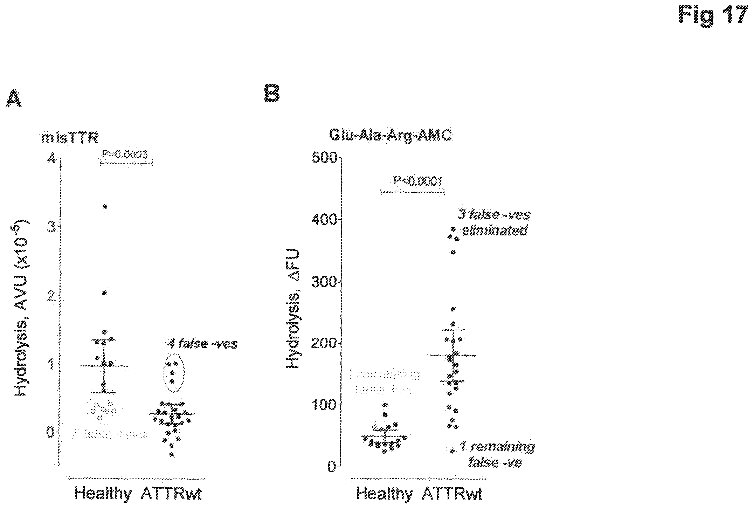

Figure 17A-17B show reduced misTTR-directed hydrolytic activity accompanied by increased polyreactive hydrolytic activity of IgM preparations from ATTRwt patients. Figure 17A shows reduced misTTR hydrolysis by IgM preparationspurified from ATTRwtpatients compared to age-matched healthy human subjects (cohort lin Table 4) determined by electrophoresis as in Example 1. IgM concentration, 100 µg/ml. False-positive healthy subjects and false-negative ATTRwt patients showed activity less than and within the mean-95% confidence limit of the healthy subject population, respectively. AVU, misTTR hydrolyzed in arbitrary electrophoresis band volume units. Figures 17B shows increased Glu-Ala-Arg-AMC (EAR-AMC) hydrolysis by the same purified IgM preparations from ATTRwtpatients compared to age-matched healthy human subjects (cohort 1 in Table 4) determined by fluorimetry as in Example 1. Applying EAR-AMC hydrolysis as a secondary test (see Table 5) eliminated 6 of 7 false-positive healthy subjects and 3 of the 4 false-negative ATTRwt patients from Figure 17A, leaving 1 false-positive healthy subject and 1 false-negative ATTRwt patient. FU, EAR-AMC hydrolyzed in fluorescence units.

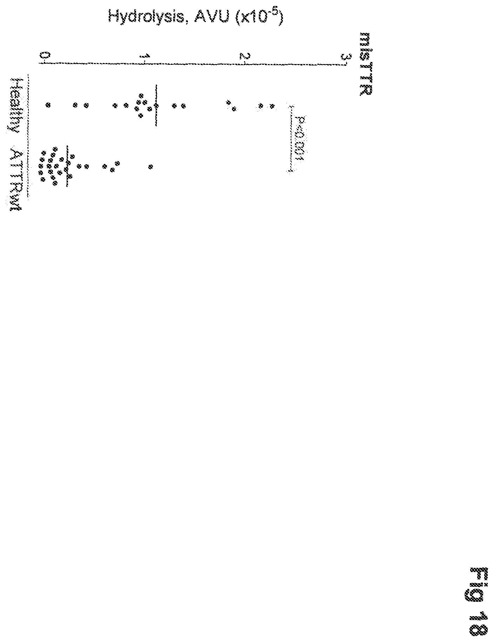

Figure 18 shows reduced misTTR-directed hydrolytic activity of unfractionated sera from ATTRwt patients. Reduced misTTR hydrolysis by unfractionated sera from ATTRwt patients compared to age-matched healthy human subjects (cohort 2 in Table 4) determined by electrophoresis as in Example 1. Serum dilution, 1:300.

### DETAILED DESCRIPTION OF THE INVENTION

This invention provides specific catalytic antibodies (catabodies) to misfolded transthyretin. The present disclosure indicates that the germline IgM repertoire is a source of catabodies with functionally useful specificity for individual amyloid substrates that has evolved over the Darwinian time frame because of the survival advantage of the amyloid clearing function [5]. Developing antibody specificity generally requires the immunogen-driven acquired immune response. Immune tolerance mostly precludes synthesis of pathogenic autoantibodies to essential self-proteins. Amyloids are pathogenic self-protein assemblies, and anti-amyloid antibodies may be beneficial. Over 27 proteins form misfolded amyloid aggregates with β-sheet character [1]. Transthyretin (TTR) is a blood-borne carrier protein for thyroid hormones and the retinol binding protein-retinol complex [2]. Soluble misfolded TTR (misTTR) aggregates formed as byproducts of alternate folding pathways of wildtype TTR act as seed for deposition of pathological TTR amyloid in the heart, lung, joints and other tissues. With advancing age, humans develop wildtype TTR amyloidosis (formal designation, ATTRwt [3]; also known as "senile systemic amyloidosis" or SSA, a disease usually occurring at age >60 years) [4]. The Val122Ile and Val130Met TTR mutations frequently cause early-onset, hereditary TTR amyloidosis (ATTRm).

Humans produce diverse antibodies by: (a) Germline transmission of multiple variable (V)-domain genes (~200 V-, D-, J-genes) attached to alternate constant (C)-domain genes (µ, δ, γ, α, ε genes; Fig1A); (b) Imprecise V-(D)-J gene recombination prior to immunogen exposure, generating the constitutive (innate) repertoire estimated at >1012 V-domains[27]; and (c) The somatic hypermutation (SHM) process introduces mutations during immunogen-driven adaptive V-domain maturation. The repertoire can be traced to cartilaginous fish that originated ~500 millions ago express the first recognizable immune system (Fig1B). There is no known extant organism with invariant V-domains, suggesting the role of diversification processes in evolutionary survival [28]. The structure of single domain antibodies in the fish is reminiscent of catalytically efficient human V domains. The fish also produce oligomer IgMs and monomer IgMs, but not IgGs.

The ontogeny of the V-domain noncovalent and proteolytic sites is quite different. The FRs contribute residues that participate directly in the catalytic mechanism. Germline-encoded VL-domains hydrolyze peptide bonds promiscuously with no requirement for high affinity epitope recognition [29]. The extensively mutated CDRs form the classical acquired immunity binding sites of IgGs [30]. The IgM C-domain scaffold improves the integrity of the V-domain catalytic site, indicated by the superior catalytic activity of the same V-domains cloned in the IgM versus IgG scaffold [31]. By definition, germline antibody activities evolved from an ancestral organism. Tracing the evolutionary origin of antibody catalysis is of interest to assess its functional importance in the establishment of immune defense systems, including defense against toxic amyloid self-proteins. There is also a practical justification. Antibodies with the greatest catalytic activity are candidate therapeutic agents. A single catalyst molecule can be reused repeatedly to clear thousands of target protein molecules. The unpaired, single domain human VL constructs with catalytic activity are structurally reminiscent of naturally-occurring single V-domain antibodies found in primordial organisms. The single domain VL constructs are described to catalyze the hydrolysis of amyloid β peptide more efficiently than paired IgG VL-VH domains [32] and single chain Fv fragments (scFv, a surrogate of the antibody binding site) [33].

Both innate and adaptive immunity factors are likely to regulate catabody production. Innate catabody synthesis in the absence of contact or stimulation by an antigen is regulated by the autonomous B cell developmental program in conjunction with numerous antigen-independent environmental factors such as cytokines [34-36]. In addition, the defined innate specificity the adaptive of B cell receptors (BCRs) for a given antigen epitope can be lost during BCR sequence maturation driven by other irrelevant antigenic epitopes. Contact of BCRs with innate specificity to a given antigenic epitope, e.g., an amyloid epitope, with that epitope itself is also conceived to regulate catabody synthesis negatively or positively. For example, excess concentrations of an amyloid epitope may induce B cell tolerance and downregulate catabody synthesis, whereas at limiting amyloid epitope concentrations, a stimulatory B cell response can occur. Knowing changes in the activity of catabodies as a function of aging is of interest. A reduction of a specific catabody activity to an individual amyloid protein may accelerate amyloidosis due to that protein, and an increase of such a catabody activity may retard amyloidosis. In addition, variations in the magnitude of the catabody activity found in different humans without clinically-evident amyloidosis may be a useful predictor for the subsequent appearance of amyloid disease. Monoclonal and polyclonal catabodies hold potential as candidate drugs against amyloidosis. Catabodies utilize nucleophilic mechanism to hydrolyze peptide bonds [30, 37]. Catabodies can be identified and their synthesis can be induced using electrophilic analogs of amyloid proteins. Non-hydrolyzable synthetic electrophilic phosphonates bond covalently with nucleophilic, IgM-class B cell receptors (BCRs) [37]. The synthetic electrophile was placed within several antigenic epitopes of Aβ and gp120, yielding the electrophilic analogs (E-Aβ, E-gp120) that were capable of binding antibodies noncovalently coordinated with covalent binding to nucleophilic sites. E-Aβ and E-gp120 were applied as probes to isolate pre-existing antibodies in the natural antibody repertoire and as immunogens to induce adaptive synthesis of nucleophilic antibodies with specific catabody activity [38, 39]. In addition, immunization with synthetic electrophilic analogs of peptides derived from gp120 induced specific antibody responses.

The functional value of catabodies to amyloids is suggested by previous studies on Aβ. Catalytic IgMs and recombinant VL constructs 5D3 and 2E6 from a human phage library attenuated Aβ oligomer neurotoxicity, reduced the formation of Aβ fibrils and oligomers, and induced disappearance of preformed Aβ aggregates in vitro [33, 40]. Clone 2E6 is a heterodimer of two VL domains. Clone 5D3 is a single domain VL. The catabody constructs hydrolyzed Aβ (His14-Gln15 bond), releasing soluble Aβ fragments [33]. Aβ binding was undetectable. Brain Aβ clearance was shown in 3 different transgenic (Tg) mouse strains expressing human Aβ. Local Aβ removal was evident after intracranially administered catalytic VL2 2E6 in aged 5XFAD mice, a model for parenchymal brain Aβ deposition [41]. Aβ plaque removal was evident after intravenous injection of VL2 2E6 in Tg-SwDI mice. These difficult-to-treat mice express mutated Aβ that forms vascular amyloid, causing microhemorrhages [42, 43]. No increase in cerebral microbleeds was observed in mice following catalyst injection, a common side-effect of Aβ-binding Abs [44-48]. Gene transfer of VL 5D3 in the brain of APPswe/PS1dE9 Tg mice resulted in prolonged expression of VL 5D3 and reduction of Aβ load in the brain. Unlike the report of cerebral microbleeds by gene transfer of an Aβ-binding antibody [49], the catalytic VL did not induce hemorrhages [50, 51].

Innate polyreactive catabodies are detected based on the ability to hydrolyze short peptide substrate without requirement for specific epitope recognition. The ability to recognize multiple, unrelated substrates might result in multiple biological functions for polyreactive catabodies depending on the homeostatic or pathological context. Importantly, the polyreactive catabody subset may fulfill a beneficial role in removal of excess target substrates that exert toxic effects [52, 53], for example, removal of excess bacterial substrates in infections, removal of excess intracellular antigens under conditions of widespread cell death and removal of excess amyloids despite low-affinity amyloid recognition. Moreover, the polyreactive catabodies may represent species with yet to be identified innate specificities directed to broad classes of senescent substrates. For the purpose of the present disclosure, a "senescent" substrate is defined as a substrate that acquires toxicity due to one or more conformational changes that generate neoepitopes absent in the physiological structure of the substrate. Such toxic neoepitopes can be induced, for example, when a non-toxic physiological substrate is subjected to post-translational chemical modifications. Similarly, neoepitopes can be induced upon release of intracellular substrates into the extracellular environment due to physicochemical devations between the extracellular and intracellular environments (e.g., redox status, concentration of various metals and chaperones). Removal of such senescent antigens by catabodies will result in beneficial effects.

The antibody of the instant invention may be a naturally occurring antibody or may be a synthetic or modified antibody (e.g., a recombinantly generated antibody; a chimeric antibody; a bispecific antibody; a humanized antibody; a camelid antibody; and the like). In a particular embodiment, the antibody is innate and naturally occurring. Once an antibody has been isolated, the sequence of the antibody can be determined by methods known in the art and the antibody can be synthesized recombinantly/synthetically.

In a particular embodiment, the antibody is an antibody fragment. Antibody fragments include, without limitation, immunoglobulin fragments including, without limitation: single domain (e.g., single variable light or heavy chain domain), Fab, Fab', F(ab')2, and Fv; and fusions (e.g., via a linker) of these immunoglobulin fragments including, without limitation: scFv, scFv2, scFv-Fc, minibody, diabody, triabody, and tetrabody. The antibody may also be a protein (e.g., a fusion protein) comprising at least one antibody or antibody fragment. The antibody may comprise at least one purification tag. The antibodies of the instant invention may also be conjugated/linked to other components. For example, the antibodies may be operably linked (e.g., covalently linked, optionally, through a linker) to at least one detectable agent, imaging agent, contrast agent, cytotoxic molecule (e.g., immunotoxin), and/or any other biological compound. In a particular embodiment of the instant invention, the antibody is a monoclonal antibody or fragment thereof.

The antibodies of the instant invention may be further modified. For example, the antibodies may be humanized. In a particular embodiment, the hybrid antibodies (or a portion thereof) are inserted into the backbone of an antibody or antibody fragment construct. For example, the variable light domain and/or variable heavy domain of the antibodies of the instant invention may be inserted into another antibody construct. Methods for recombinantly producing antibodies are well-known in the art. Indeed, commercial vectors for certain antibody and antibody fragment constructs are available. The purity of the antibody molecules of the invention may be assessed using standard methods known to those of skill in the art, including, but not limited to, ELISA, immunohistochemistry, ion-exchange chromatography, affinity chromatography, immobilized metal affinity chromatography (IMAC), size exclusion chromatography, polyacrylamide gel electrophoresis (PAGE), western blotting, surface plasmon resonance and mass spectroscopy.

The antibodies or the electrophilic analog of the instant invention ((or composition(s) comprising the same (e.g., with at least one pharmaceutically acceptable carrier)) can be administered by any suitable route, for example, by injection (e.g., for local, direct, or systemic administration), oral, pulmonary, topical, nasal or other modes of administration. The composition may be administered by any suitable means, including parenteral, intramuscular, intravenous, intravascular, intraarterial, intraperitoneal, subcutaneous, topical, inhalatory, transdermal, intrapulmonary, intraareterial, intrarectal, intramuscular, and intranasal administration. In a particular embodiment, the composition is administered to the blood. In general, the pharmaceutically acceptable carrier of the composition is selected from the group of diluents, preservatives, solubilizers, emulsifiers, adjuvants and/or carriers. The compositions can include diluents of various buffer content (e.g., Tris HCl, acetate, phosphate), pH and ionic strength; and additives such as detergents and solubilizing agents (e.g., Polysorbate 80), anti oxidants (e.g., ascorbic acid, sodium metabisulfite), preservatives (e.g., Thimersol, benzyl alcohol) and bulking substances (e.g., lactose, mannitol). The compositions can also be incorporated into particulate preparations of polymeric compounds such as polyesters, polyamino acids, hydrogels, polylactide/glycolide copolymers, ethylenevinylacetate copolymers, polylactic acid, polyglycolic acid, etc., or into liposomes. Such compositions may influence the physical state, stability, rate of in vivo release, and rate of in vivo clearance of components of a pharmaceutical composition (see, e.g., Remington's Pharmaceutical Sciences and Remington: The Science and Practice of Pharmacy). The pharmaceutical composition can be prepared, for example, in liquid form, or can be in dried powder form (e.g., lyophilized for later reconstitution).

The therapeutic agents described herein will generally be administered to a patient as a pharmaceutical preparation. The term "patient" as used herein refers to human or animal subjects. The compositions may be employed therapeutically or prophylactically, under the guidance of a physician.

Compositions comprising the agent may be conveniently formulated for administration with any pharmaceutically acceptable carrier(s). The concentration of agent in the chosen medium may be varied and the medium may be chosen based on the desired route of administration of the pharmaceutical preparation. Except insofar as any conventional media or agent is incompatible with the agent to be administered, its use in the pharmaceutical preparation is contemplated.

The dose and dosage regimen of the agent that is suitable for administration to a particular patient may be determined by a physician considering the patient's age, sex, weight, general medical condition, and the specific condition for which the agent is being administered to be treated or prevented and the severity thereof. The physician may also take into account the route of administration, the pharmaceutical carrier, and the agent's biological activity. Selection of a suitable pharmaceutical preparation will also depend upon the mode of administration chosen.

A pharmaceutical preparation may be formulated in dosage unit form for ease of administration and uniformity of dosage. Dosage unit form, as used herein, refers to a physically discrete unit of the pharmaceutical preparation appropriate for the patient undergoing treatment or prevention therapy. Each dosage should contain a quantity of active ingredient calculated to produce the desired effect in association with the selected pharmaceutical carrier. Procedures for determining the appropriate dosage unit are well known to those skilled in the art.

Dosage units may be proportionately increased or decreased based on the weight of the patient. Appropriate concentrations for alleviation or prevention of a particular condition may be determined by dosage concentration curve calculations, as known in the art.

The pharmaceutical preparation comprising the agent may be administered at appropriate intervals until the pathological symptoms are reduced or alleviated, after which the dosage may be reduced to a maintenance level. The appropriate interval in a particular case would normally depend on the condition of the patient.

Toxicity and efficacy (e.g., therapeutic, preventative) of the particular formulas described herein can be determined by standard pharmaceutical procedures such as, without limitation, in vitro, in cell cultures, ex vivo, or on experimental animals. The data obtained from these studies can be used in formulating a range of dosage for use in human. The dosage may vary depending upon form and route of administration. Dosage amount and interval may be adjusted individually to levels of the active ingredient which are sufficient to deliver a therapeutically or prophylactically effective amount.

### Definitions

The following definitions are provided to facilitate an understanding of the present disclosure:
The term "isolated" may refer to a compound or complex that has been sufficiently separated from other compounds with which it would naturally be associated. "Isolated" is not meant to exclude artificial or synthetic mixtures with other compounds or materials, or the presence of impurities that do not interfere with fundamental activity or ensuing assays, and that may be present, for example, due to incomplete purification, or the addition of stabilizers.

"Pharmaceutically acceptable" indicates approval by a regulatory agency of the Federal or a state government or listed in the U.S. Pharmacopeia or other generally recognized pharmacopeia for use in animals, and more particularly in humans.

A "carrier" refers to, for example, a diluent, adjuvant, preservative (e.g., benzyl alcohol), anti-oxidant (e.g., ascorbic acid, sodium metabisulfite), solubilizer (e.g., Tween 80, Polysorbate 80), emulsifier, buffer (e.g., Tris HCl, acetate, phosphate), bulking substance (e.g., lactose, mannitol), excipient, auxilliary agent, filler, disintegrant, lubricating agent, binder, stabilizer, preservative or vehicle with which an active agent is administered. Pharmaceutically acceptable carriers can be sterile liquids, such as water and oils, including those of petroleum, animal, vegetable or synthetic origin, such as peanut oil, soybean oil, mineral oil, sesame oil and the like. Water or aqueous saline solutions and aqueous dextrose and glycerol solutions are preferably employed as carriers, particularly for injectable solutions. The pharmaceutical composition can be prepared, for example, in liquid form, or can be in dried powder form (e.g., lyophilized). Suitable pharmaceutical carriers are described, for example, in "Remington's Pharmaceutical Sciences" by E.W. Martin (Mack Publishing Co., Easton, PA); Gennaro, A. R., Remington: The Science and Practice of Pharmacy, (Lippincott, Williams and Wilkins); Liberman, et al., Eds., Pharmaceutical Dosage Forms, Marcel Decker, New York, N.Y.; and Kibbe, et al., Eds., Handbook of Pharmaceutical Excipients, American Pharmaceutical Association, Washington.

An "antibody" or "antibody molecule" is any immunoglobulin, including antibodies and fragments thereof, that binds to a specific antigen. As used herein, antibody or antibody molecule contemplates intact immunoglobulin molecules, immunologically active portions of an immunoglobulin molecule, and fusions of immunologically active portions of an immunoglobulin molecule. Antibody fragments include, without limitation, immunoglobulin fragments including, without limitation: single domain (e.g., single variable light or heavy chain domain), Fab, Fab', F(ab')2, and Fv; and fusions (e.g., via a linker) of these immunoglobulin fragments including, without limitation: scFv, scFv2, scFv-Fc, minibody, diabody, triabody, and tetrabody.

With respect to antibodies, the term "immunologically specific" refers to antibodies that bind to one or more epitopes of a protein or compound of interest, but which do not substantially recognize and bind other molecules in a sample containing a mixed population of antigenic biological molecules.

The singular forms "a," "an," and "the" include plural referents unless the context clearly dictates otherwise.

As used herein, a "biological sample" refers to a sample of biological material obtained from a subject, preferably a human subject, including a tissue, a tissue sample, a cell sample, a protein sample, a DNA sample, an RNA sample and a biological fluid (e.g., blood, serum, plasma, etc.). In a particular embodiment, the biological sample is blood.

As used herein, "diagnose" refers to detecting and identifying a disease or disorder in a subject. The term may also encompass assessing or evaluating the disease or disorder status (progression, regression, stabilization, response to treatment, etc.) in a patient and/or providing a prognosis of the disease or disorder.

As used herein, the term "prognosis" refers to providing information regarding the impact of the presence of a disease or disorder (e.g., as determined by the diagnostic methods of the present invention) on a subject's future health (e.g., expected morbidity or mortality, the likelihood of getting diabetes, and the risk of cardiovascular disease). In other words, the term "prognosis" refers to providing a prediction of the probable course and outcome of a disease/disorder or the likelihood of recovery from the disease/disorder.

The term "treat" as used herein refers to any type of treatment that imparts a benefit to a patient afflicted with a disease or disorder, including improvement in the condition of the patient (e.g., in one or more symptoms), delay in the progression of the condition, etc.

The following examples are provided to illustrate certain embodiments of the invention. They are not intended to limit the invention in any way.

### EXAMPLE 1: PHYSIOLOGICAL IgM CLASS CATALYTIC ANTIBODIES SELECTIVE FOR TRANSTHYRETIN AMYLOID

Higher organisms possess a structurally diverse innate repertoire of antibody variable (V) domains that was shaped by evolutionary selection pressures imposed by foreign antigens and self-antigens over millions of years. The diversity is supplemented by somatic randomization of the V-domains by acquired immunity processes, which enables selection of V-domains with noncovalent antigen binding activity over a few weeks upon contact with an antigen. Polyreactive catalytic antibodies (catabodies) with a promiscuous peptide bond hydrolytic activity, on the other hand, are produced as an innate property of B cells, requiring no exposure to the antigen[37, 56]. The proteolytic activity was traced to serine protease-like sites in the germline antibody V-regions, suggesting an ancient origin of the catabodies [29, 57-59]. Humans produce catabodies selective for amyloid β peptide (Aβ)[55]. Immune tolerance mechanisms in fetal and adult life are thought to suppress synthesis of autoantibodies to essential self-proteins. When produced, subsets of autoantibodies express autoantigen-selective catalytic activities [40, 60, 61]. Provided catabodies discriminate between individual peptide antigens, they hold potential for specific and permanent destruction of toxic self-antigens.

Many proteins can misfold into the amyloid state [1]. Soluble transthyretin (TTR) is found in large amounts in blood (-0.3 mg/mL) in its physiological, homotetrameric state (phyTTR) as a carrier protein for thyroid hormones and the retinol-retinol binding protein complex [2]. Misfolded β-sheet containing aggregates are generated from TTR (collectively designated misTTR states, Table 1), a process involving formation of a TTR monomer, destabilization of the phyTTR dimer-dimer interface, and growth of soluble misTTR oligomers into fibrillar amyloid[62-64]. Small misTTR amounts are likely formed continuously as basal byproducts of alternate TTR folding pathways. Initial evidence for the existence of blood-borne misTTR species is available [65]. Dysregulated protein misfolding during normal aging processes causes increased TTR amyloid deposition in several tissues [2]. TTR amyloid deposition in the tenosynovium is associated with carpal tunnel syndrome in middle-aged humans [66]. TTR amyloidosis in the heart, lung, stomach, kidney and other tissues causes organ failure in ATTRwt, a disease affecting > 10% humans over age 70 years [2, 4, 16, 67]. In addition, mutations in the TTR gene cause early-onset familial TTR amyloidosis [68]. Amyloid deposits cause architectural and functional tissue damage, and soluble misTTR aggregates exert direct cytotoxic effects. No approved pharmacological treatment for ATTRwt or ATTRm is available, but anti-transthyretin RNAi reagents are undergoing clinical trials [24] and a small molecule drug that interacts with the thyroxine binding site of TTR may help relieve symptoms of polyneuropathy in familial TTR amyloidosis [22]. Reversibly-binding antibodies that can clear amyloids by phagocytosis of the immune complexes were proposed as potential therapeutic reagents [48, 69, 70]. IgM class catabodies produced by healthy humans that hydrolyze misTTR but not phyTTR are described. The catabodies can be conceived as homeostatic mediators that destroy TTR amyloid and its precursor misfolded TTR forms without interfering in the physiological functions of TTR.

**Table 1.Properties of physiological TTR (phyTTR) and misfolded TTR (misTTR). ISF: interstitial fluid. ^{a}, refs [71-73].**

| ***Property*** | ***phyTTR*** | ***misTTR*** |
|---|---|---|
| Solubility | Soluble | Particulate and soluble |
| Aggregate subunit composition | 4mer | Variable (2 - millions) |
| Aggregate stability in boiling SDS | Dissociate into 1mers | Dissociate into 1mers |
| Aggregate stability in SDS without boiling | Remain as 4mers^{a} | Dissociate into 1mers^{a} |
| Biological distribution | Blood, tissue ISF | Blood, tissue ISF, tissue amyloid |
| Pathogenicity | None | Cellular toxicity, anatomic disruption |

### Materials and Methods

Antibodies. Human studies were approved by the Committee for the Protection of Human Subjects, University of Texas Health Science Center, Houston. Individual or pooled polyclonal IgM, IgG and IgA class antibodies were purified from sera of 12 healthy humans without amyloidosis or autoimmune disease (33±7 years age) by acid elution (pH 2.7) from columns of immobilized anti-human IgM antibodies, Protein G and anti-human IgA antibodies [37, 74]. The antibody preparations were free of detectable non-antibody proteins judged by SDS-electrophoresis and immunoblotting with specific antibodies to IgM, IgG and IgA.For evaluation of aging effects, IgMs were purified from non-aged humans (<35 years age) or aged humans (>70 years) of either gender without amyloidosis or autoimmune disease. The monoclonal IgM (mIgM) panel purified from patients with Waldenström's macroglobulinemia patients was described (our laboratory codes 1800-1804, 1806, 1809-1811, 1813, 1814, 1816-1819, Yvo) [56]. The panel is assumed to be a random collection of mIgMs. There is no known relationship between B cell carcinogenicity and IgM catalytic activity or antigenic specificity. Unfractionated human serum was pooled from 10 healthy humans (36±5 years age). To prepare antibody-free serum, the pooled serum was adsorbed sequentially on the anti-IgM, Protein G and anti-IgA columns (residual IgM, IgA and IgG estimated by ELISA were, respectively, 0.05, 0.007 and 0.003%) [31]. FPLC gel filtration of the unfractionated serum pool (70 µL) was on a Superose-6 column (GE Healthcare, Piscataway, NJ; flow rate 0.1 mL/min) in 10 mM sodium phosphate, pH 7.4, 137 mM NaCl, 2.7 mM KCl (PBS) containing 0.1 mM CHAPS. The Aβ-hydrolyzing recombinant catalytic antibody fragment (clone 2E6) was purified as described [33]. Nominal molecular mass was computed by comparison with protein markers (a reference 900 kDa mIgM and 1.4-670 kDa markers from Biorad, Hercules, CA). Total protein was measured by the microBCA method (ThermoFisher Scientific, Rockford, IL).Cell-surface IgMs were analyzed using the purified peripheral blood B cells from a 25 year old human subject without amyloidosis (B cell negative selection kit, Miltenyi, Auburn, CA; viability 90-95%; >95% purity determined by staining with phycoerythrin-conjugated mouse antibody to human CD19 (BD Pharmingen, San Jose, CA) and flow cytometry.

Aggregated TTR. Wildtype, purified TTR from human plasma (Cell Sciences, Canton, MA) was labeled with ¹²⁵I (¹²⁵I-TTR) using 1,3,4,6-tetrachloro-3α,6α-diphenylglycouril(ThermoFisher), followed by removal of free ¹²⁵I by gel filtration (BioSpin-6 column; Bio-Rad). The TTR or ¹²⁵I-TTR solutions were pre-aggregated in PBS containing 1 mM EDTA (0.4 mg TTR/mL, 28.6 µM; molar TTR concentrations computed from the monomer TTR mass, 14 kDa) by acidification with an equal volume of 200 mM sodium acetate, pH 4.2, 100 mM KCl, 1 mM EDTA, followed by incubation for 5 days at 37°C [64]. After exchanging the buffer to PBS containing 0.1 mM CHAPS (PBS/CHAPS) with an Ultra-4 centrifugal filter (EMD Millipore, Billerica, MA), the aggregated TTR (~ 14 µM) was stored in aliquots at -80°C. Non-aggregated TTR is composed of soluble physiological tetramers, and the aggregation reaction produces soluble and particulate misfolded TTR [62-64]. Aggregation was monitored by turbidimetry at 400 nm (10 mm path length; Cary 50 spectrophotometer, Agilent, Santa Clara, CA). In addition binding of TTR (100 µL, 0.1 mg/mL) to thioflavin T (ThT) was determined by mixing with ThT (2.5 µL, 0.6 mM in PBS containing 0.1 mM CHAPS and 12% dimethylsulfoxide) and measurement of fluorescence emission (λₑₘ 485 nm, λₑₓ 440 nm, 600 V PMT voltage; Varian Cary Eclipse fluorimeter). The supernatant and pellet containing the soluble and particulate TTR species, respectively, were separated by centrifugation (17,000 x g, 20 min).

The phyTTR and misTTR states were dissociated into monomers (14 kDa) by boiling test samples (10 min) after mixing with a 1/5th volume of 10% SDS in electrophoresis buffer (0.313 M Tris-HCl, pH 6.8, containing 17% 2-mercaptoethanol, 50% glycerol and 0.025% Bromophenol Blue). Electrophoresis was in 16.5% polyacrylamide Criterion gels (Bio-Rad) in 100 mM Tris-Tricine, pH 8.3, containing 0.1% SDS. Protein mass was computed by comparison with standard proteins (14-94 and 1.4-26.6 kDa ladders; Biorad). The phyTTR and misTTR states were distinguished by electrophoresing non-boiled test samples treated with 10% SDS treatment in non-reducing buffer. The 54 kDa phyTTR 4mers dissociate into 1mers by upon boiling in SDS but remain undissociated in SDS at room temperature, whereas SDS treatment at room temperature is sufficient to dissociate misTTR aggregates into 14 kDa 1mers [71-73]. Radioactive TTR bands were detected with a Cyclone Plus Phosphor Imager (PerkinElmer, Waltham, MA; 30-120 min exposure, super-sensitive storage screen; minimum detectable radioactivity+3 s.d. values, 500 CPM; linear detection up to 867,000 CPM). % misTTR content of pre-aggregated ¹²⁵I-TTR was determined by densitometry as: 100 x [14 kDa band intensity]/[sum of 14 and 54 kDa band intensities], where the band intensity was in arbitrary volume units (AVU; ImageJ software, NIH Research Services Branch, Bethesda, MD).

The biotinylated, electrophilic analogs of misTTR (E-misTTR) and phyTTR (E-phyTTR) were prepared as described [39](6.0 and 0.1 mol phosphonate and biotin, respectively, per molmisTTR; 3.9 and 1.5 mol phosphonate groups and biotin, respectively, per mol phyTTR). The starting material for E-misTTR preparation was the particulate misTTR in PBS/CHAPS recovered by centrifugation (30 min, 75,000g).

Proteolysis. All tests were done in duplicate or as specified. Non-aggregated or pre-aggregated ¹²⁵I-TTR (100 nM monomer equivalents) was incubated with purified antibodies in PBS/CHAPS (20 µL). The boiled or non-boiled reaction mixtures were analyzed by SDS-electrophoresis (CPM/lane, 8,930-17,110). Depletion of the 1mer TTR band (14 kDa) and appearance of lower mass products was quantified by densitometry. Antibody hydrolytic activity was computed in units of nM misTTR hydrolyzed/µg antibody/hour ((AnM x (1 - [14 kDaAb]/[14 kDa_{Dil}]))/(µg Ab in the reaction mixture/Ab treatment time in hours), where: A is the initial misTTR concentration (total TTR concentration x % misTTR content) if non-boiled samples were analyzed or the total TTR concentration (misTTR+phyTTR) if boiled samples were analyzed; Ab denotes antibody; and, [14 kDaAb] and [14 kDaDil] are the 14 kDa band intensities after antibody and diluent treatment, respectively. For data reported in this format, variations of the misTTR hydrolysis rate up to 2.4-fold are predicted because of varying misTTR content in different pre-aggregated TTR preparations (29-70%).

Hydrolysis of non-radiolabeled TTR bands stained with silver was quantified similarly. Protease inhibitors were tested at concentrations sufficient for complete inhibition of metalloproteases (EDTA, 2 mM; 1,10-phenanthroline, 1 mM), cysteine proteases (iodoacetamide, 0.1 mM), acid proteases (pepstatin A, 1 µM) and serine proteases (E-hapten 1, a small molecule phosphonatediester, 0.1 mM)[75]. E-hapten 1 ((diphenylN-[6-(biotinamido)hexanoyl]amino(4-amidinophenyl)methane phosphonate)) is an active site directed inhibitor of nucleophilic catabodies [76]. Alkylation of the free -SH group in TTR was avoided by pre-treating antibodies with iodoacetamide (60 min) and removing this reagent prior to the proteolysis assay (BioSpin-6 gel filtration column). Inhibition of antibody proteolysis by serum amyloid P component (SAP; Calbiochem, Billerica, MA) was tested under conditions reported to reduce TTR sensitivity to digestion by human matrix metalloproteinase-9 (MMP9 from human neutrophils, EMD Millipore; pre-aggregated ¹²⁵I-TTR containing 1 µM misTTR pre-treated with 0.55 µM SAP or control albumin for 2 h in 10 mM Tris HCl, pH 8.0, 138 mM NaCl, 2 mM CaCl₂) [77]. MMP9 was activated by incubation for 3 h with 5 mM 4-aminophenylmercuric acetate, and the enzyme was recovered by gel filtration for hydrolysis tests.

Hydrolysis of fluorigenic EAR-AMC (N-t-butoxycarbonyl-O-benzyl-Glu-Ala-Arg-7-amino-4-methylcoumarin) and amyloid β peptide 1-40 (Aβ1-40) was tested as in ref [55]. ¹²⁵I-Aβ 1-42 (Aβ42, 90 µg, EZBiolab, Carmel, IN) was radiolabeled as described for TTR, and free ¹²⁵I was removed using a Sep-Pak Vac C18 cartridge [78]. The fibrillar Aβ42 substrate (2.3 x 107 CPM/µmol Aβ42) was prepared by co-aggregating ¹²⁵I-Aβ42 (0.7x10⁹ CPM in 1,1,1,3,3,3-hexafluoro-2-propanol, 0.1 mL) and Aβ42 (1 mg; 5 mM solution in dimethylsulfoxide). The mixture was diluted to 50 µM Aβ42 with PBS, incubated for 92 h (37°C), and the fibrillar ¹²⁵I-Aβ42 was collected by centrifugation (17,000xg, 20 min). Following digestion of fibrillar ¹²⁵I-Aβ1-42 with antibodies (30,000 CPM, 16 h), the reaction mixtures were treated with formic acid (60%v/v) and fractionated on a Superdex peptide gel filtration column (GE Healthcare) in 60% formic acid. Hydrolysis of these biotinylated proteins was measured by SDS-electrophoresis and staining with streptavidin-peroxidase as in ref [56](molar biotin/protein ratio in parentheses): gp120 from HIV strain MN (1.6), S. *aureus* Protein A (5.0), bovine serum albumin (9.0), extracellular domain of human epidermal growth factor receptor (2.3), bovine thyroglobulin (26.0), human transferrin (5.4), and ovalbumin (1.6).

Binding. Pre-aggregated ¹²⁵I-TTR (25,000 cpm; 100 nM TTR, 60% misTTR content) was incubated (16 h) with or without IgMs (130 µg/mL) in 40 µL in PBS/CHAPS. The soluble and particulate fractions were separated by centrifugation (20 min, 17,000g), the particulate fraction was resuspended in 40 µL PBS/CHAPS, treated separately for 1 h with the immobilized anti-human IgM gel (50 µL settled gel), the mixtures were centrifuged (5min, 500g), the gel was washed with 250 µL PBS/CHAPS, and radioactivity in supernatant (unbound ¹²⁵I-TTR) and gel (bound ¹²⁵I-TTR) was measured. % bound ¹²⁵I-TTR was computed as 100 X (CPM total binding - CPM nonspecific binding)/CPM ¹²⁵I-TTR in the initial reaction mixture), where total binding refers to the binding in IgM containing reaction mixtures and nonspecific binding refers to binding in the reactions containing PBS/CHAPS instead of IgMs.

For cell binding studies, large E-misTTR particles were removed by centrifugation (400g, 10 min), and the E-misTTR did not contain flow cytometrically detectable particles. B cells were pretreated with mouse serum (1% in PBS, 10 min) to saturate Fc-receptors and with Endogenous Avidin/Biotin Blocking kit to saturate cellular biotin as recommended by the manufacturer (Abcam, Cambridge, MA). The cells (1.5 x10⁵) were incubated with E-phyTTR or E-misTTR (0.3 µM) in PBS for 1h at 4°C to minimize B cell receptor internalization (BCR).Competition studies were done by preincubating B cells (1 h, 4°C) with control diluent or non-biotinylated misTTR, phyTTR or ovalbumin (3 µM). Bound biotinylated probes were detected with streptavidin conjugated to phycoerythrin-Cy7 (PECy7-SA; 8 µg/mL). IgM+ cells were identified with antibody to human IgM conjugated to fluorescein isothiocyanate(FITC; 2 µg/mL, BDPharmingen). Flow cytometry was done using an LSRFortessa X-20 flow cytometer (BD Biosciences, San Jose, CA) and FlowJo software(about 10,000 cells/analysis). In control incubations, B cells were stained with FITC-labeled, isotype-matched antibody instead of the anti-IgM antibody or were treated with diluent instead of the biotinylated E-misTTR followed by staining with PECy7-SA.The E-misTTR binding cells were visualized by confocal microscopyas in[37](multiple acquisitions, thickness 0.15 µm; deconvolution by 15 iterations; FITC - λₑₓ 488nm, λₑₘ 525nm; 4,6 diamino-2-phenylindole - λₑₓ 350nm, λₑₘ 470nm; PECy7 - λₑₓ 488 nm, λₑₘ 767nm).

Amyloid Dissolution. Pre-aggregated non-radioactive TTR (7.1 µM, 100 µg/mL) was incubated in duplicate with diluent or mIgMs (120 µg/mL) in PBS containing 0.1 mM CHAPS at 37°C. The dissolution reaction was monitored from turbidity or ThT binding measurements.

Statistical analysis. Errors are s.d. values. P values were from the unpaired two-tailed Student's t-test. Correlations were determined by the Pearson's two-tailed test.

### Results

¹²⁵I-TTR substrate. Wildtype human TTR radiolabeled with ¹²⁵I (¹²⁵I-TTR) was applied to determine hydrolysis of the phyTTR tetramers and misTTR aggregates. Non-aggregated ¹²⁵I-TTR preparations behaved as predicted from studies on phyTTR tetramers[71-73]. The non-aggregated ¹²⁵I-TTR: (a) did not bind appreciably to Thioflavin-T (ThT; Fig. 2A), a probe for β-sheet structures; (b) formed minimal particulate aggregates detected by turbidimetry (Fig.2B); and (c) dissociated from the 4mer into the 1mer state upon boiling in SDS(14 kDa band in lane 1, Fig2C; <1% tetramer content) but remained in the 4mer 54 kDa state in non-boiled SDS solutions (lane 2, Fig. 2C; <1% 1mer content). Incubation of ¹²⁵I-TTR at acid pH generated misTTR aggregates that bound ThT and formed a turbid suspension(Fig. 2A,2B). Unlike the 4mer phyTTR state, the misTTR aggregates were dissociated into 1mersin SDS without prior boiling (compare lanes1 and 3, Fig. 2C). The relative 4mer/1mer band intensity in non-boiled, pre-aggregated ¹²⁵I-TTR, therefore, is an index of the phyTTR/misTTR ratio (misTTR content of 6 pre-aggregated ¹²⁵I-TTR preparations from densitometry, 49±15%). The particulate fraction recovered from a pre-aggregated ¹²⁵I-TTR preparation (-50% of total radioactivity, Fig. 2D) was composed almost exclusively of misTTR species (>95% 1mers in lane 2, Fig2E). The 1mers were present at lower levels in the supernatant (soluble fraction) of pre-aggregated but not non-aggregated TTR (compare lane 3, Fig. 2D with lane 1,Fig. 2C), consistent with the existence of soluble misTTR states[ 11, 79]. Small radioactivity amounts were found at positions corresponding to the TTR dimer (nominal mass 32 kDa) and unidentified impurities in the starting TTR (20 and 26 kDa). ThT fluorescence and turbidity measurements indicated comparable aggregation of ¹²⁵I-TTR and non-radiolabeled TTR, suggesting unaltered aggregation properties of the radiolabeled protein. The results show that the phyTTR and misTTR states are distinguishable by electrophoresis.

misTTR but not phyTTR hydrolysis by polyclonal IgMs (pIgMs).The hydrolytic activity of electrophoretically homogeneous polyclonal IgM, IgG and IgA pooled from the blood of humans without TTR amyloidosis was tested. The pIgMs hydrolyzed pre-aggregated ¹²⁵I-TTR, indicated by depletion of thelmer TTR band (14 kDa) in SDS-treated, boiled reaction mixtures (Fig. 3A). The hydrolytic activity was detected in every individual pIgM preparation from 12 healthy humans(Table 2) but not IgG or IgA from the same humans. Treatment of non-aggregated ¹²⁵I-TTR with the pIgMs did not deplete the 1mer band derived from phyTTR in boiled reaction mixtures, indicating that the pIgMs do not degrade phyTTR. Depletion of the 1mer band in boiled reaction mixtures of the pre-aggregated substrate, therefore, measures misTTR hydrolysis without interference from phyTTR hydrolysis. The pIgMs also degraded pre-aggregated TTR without the ¹²⁵I label (Fig. 3B), eliminating radioactivity-related experimental artifacts. Selective recognition of soluble and particulate misTTR species by the IgM was confirmed in tests of non-boiled reaction mixtures. phyTTR does not dissociate into 1mers without boiling, and the 1mer band is derived exclusively from the misTTR species. The 1mer band derived from misTTR but not the 4mer phyTTR band was progressively depleted with increasing duration of pIgM treatment (Fig. 3C, 3D). Product fragments with mass smaller than the 1mer TTR band were evident (13 and 10 kDa bands, along with the fainter 7 kDa band, Fig. 3A).Accumulation of the 10 kDa product in the initial reaction phase and its subsequent disappearance with further pIgM treatment suggested reuse of this misTTR fragment for further digestion cycles (Fig. 3D). Appearance of 3 radioactive products implies hydrolysis of at least two peptide bonds. As the radioactivity from a single intact TTR molecule is distributed into multiple products, the product bands are less intense than the intact TTR band. The assay method does not detect small products that migrate out of the gel or products that lack a radiolabeled Tyr residue.

**Table 2. 1mer TTR hydrolytic activity of IgMs.IgM preparations with hydrolytic activity >0.1 nM/h/µg were designated 'positive'. Polyclonal IgMs were from non-aged humans (<35 years). The hydrolytic activities of IgMs from male and female humans were statistically indistinguishable (n=9 and 3, respectively; P>0.05). Monoclonal IgMs were from patients with Waldenström's macroglobulinemia.**

| | Number of IgMs | | Hydrolytic activity, nM/h/µg IgM | | | |
|---|---|---|---|---|---|---|
| | Tested | Positive | Range | Mean | SD | Median |
| Polyclonal IgMs | 12 | 12 | 0.40-1.64 | 0.93 | 0.39 | 0.98 |
| Monoclonal IgMs | 16 | 12 | 0.16-2.98 | 1.51 | 1.07 | 1.47 |

Formation of IgM-misTTR immune complexes by the pooled pIgM (130 µg/ml) was undetectable (binding of soluble and particulate misTTR species, respectively: 1.9±2.4 and 0.7±0.7%). At an equivalent concentration, the same pIgM preparation hydrolyzed 45±1% soluble misTTR and 77±1% particulate misTTR, respectively. The results indicate hydrolysis of misTTR but not phyTTR by IgMs, the first class of antibodies produced by B cells. In contrast, the IgGs and IgAs usually associated with acquired immune responses were devoid of misTTR hydrolytic activity.

Polyclonal IgM hydrolytic properties. Mean misTTR hydrolysis by pIgM preparations from humans >70 years age was only slightly lower than humans <35 years age, and the difference did not reach statistical significance (P=0.101; Fig4A). misTTR hydrolysis was detected at low pIgM concentrations (10 µg IgM/mL, approximately 200-fold lower than the blood IgM concentration). The pIgM hydrolytic rate was comparable to metalloproteinase-9 (MMP-9,Fig. 4B), an enzyme suggested to limit tissue deposition of TTR amyloid[77]. The blood IgM concentration, however, exceeds that of MMP-9 (3.2 nM) and other non-IgM proteases (<1 nM) by about 3 log orders [80, 81]. misTTR is generated from wildtype phyTTR slowly at physiological pH [82], and saturation of the IgM by trace misTTR amounts in blood[65] is unlikely. From the observed rates, 50% degradation of 1 nM misTTR at the blood IgM and MMP-9 concentrations is predicted to occur in 24 min and 28 days, respectively. Additional findings supported an important role of the IgM: (a) The hydrolytic activity of unfractionated human serum was lost nearly completely upon antibody removal by immunoadsorption (Fig. 4C); (b) The TTR product fragment profiles for purified pIgMs and unfractionated serum were identical (13, 10 and 7 kDa bands; inset, Fig. 4C); and (c) Only the 900 kDa pIgM fraction separated from other serum components by FPLC gel filtration expressed hydrolytic activity (Fig. 4D).

pIgM catalyzed misTTR hydrolysis was inhibited by synthetic E-hapten 1, a compound that binds covalently to the nucleophilic sites of serine proteases (Fig. 4E). Synthetic inhibitors of metalloproteases, acid proteases and cysteine proteases were ineffective. The inhibitor profile is consistent with findings that catabodies use a nucleophilic proteolytic mechanism [30, 37].Non-antibody proteases are sensitive to endogenous enzyme inhibitors present in serum, and the enzymatic reaction may also be inhibited by amyloid binding proteins, e.g., SAP [77]. Including antibody-free human serum or purified SAP in the reaction mixtures did not reduce the misTTR hydrolytic activity of pIgM (compare with control albumin treatment, Fig. 4E). Moreover, as misTTR was hydrolyzed by unfractionated IgMs without removal of any serum components, the IgMs are insensitive to serum protease inhibitors. One cannot exclude a serum cofactor that enhances IgM catalysis, as the hydrolytic activity of unfractionated serum per unit IgM mass exceeded that of pure pIgM from the same serum by 4.5 fold (respectively, 7.2 and 1.6 nM/µg IgM/h; this is a valid comparison because nearly all of the serum hydrolytic activity is due to the IgMs). Catabody-activating cofactors were described previously [83, 84]. Alternatively, as catabodies are sensitive to conformational perturbations [33], the acid elution procedure employed for IgM purification may attenuate the hydrolytic activity.

**Table 3: Apparent kinetic parameters for hydrolysis of pre-aggregated TTR by polyclonal IgM. Kₘ is reported as µM substrate. Vₘₐₓ is reported as µM substrate hydrolyzed over 5 days (IgM half-life in blood) at the IgM concentration in blood (2 mg/ml). Values are from hydrolytic rates at increasing concentration of pre-aggregated ¹²⁵I-TTR fitted to the Michaelis-Menten equation (160 nM-14.4 µM, misTTR content 60%; r² 0.94). IgM, 70 µg/ml.**

| **Substrate** | **Kₘ** | **Vₘₐₓ** |
|---|---|---|
| Pre-aggregated TTR | 1.3±0.3 | 269.3±15.8 |
| EAR-AMC | 120±11 | 44,352±1,584 |

The pIgM hydrolytic activity was saturable with increasing misTTR concentration (Table 3). In addition to misTTR, the pIgMs hydrolyzed small peptide substrates, a reaction that reports the catalytic subsite properties independent of noncovalent epitope binding[37, 52].The apparent Km for misTTR was 92-fold lower than Glu-Ala-Arg-AMC (EAR-AMC), a small peptide hydrolyzed by IgMs at the Arg-AMC bond, suggesting selective IgM-misTTR recognition (Km approximates Kd, the equilibrium dissociation constant). Diffusional restrictions known to decelerate enzymatic conversion of particulate substrates compared to soluble substrates [85, 86] likely limit the rate of particulate misTTR hydrolysis by the IgMs. The pIgM preparations are a mixture of individual IgM subsets, and misTTR is a mixture of multiple misTTR aggregation states. The apparent kinetic constants, therefore, are average values for hydrolysis of multiple misTTR species by multiple IgMs.

misTTR-directed B cells and monoclonal IgMs (mIgMs). The reactivity of mIgMs expressed as BCRs in healthy humans often indicates initial antibody specificity prior to contact with external immunogens. The E-misTTR analog contains electrophilic phosphonates that bind covalently to nucleophilic BCR sites responsible for catalysis, permitting identification of misTTR-selective B cells without loss of the bound probe due to peptide bound hydrolysis. Flow cytometry identified a discrete IgM+ B cell population with saturable binding activity for E-misTTR that was inhibited by competitor misTTR but not the irrelevant protein ovalbumin (Fig.5A). The intensity of E-misTTR staining differed over 2 log orders, suggesting the variable E-misTTR reactivity of individual B cells. Saturable E-phyTTR binding by the B cells was negligible, indicating selective recognition of the E-misTTR probe (Fig. 5B). Most of the E-misTTR binding sites were colocalized with cell surface IgM, consistent with BCRs recognition of the misTTR (Fig5C, image 3 is a merged rendition).

A panel of mIgMs secreted by cancerous B cells from patients with Waldenström's macroglobulinemia without TTR amyloidosis was applied as a model for individual IgMs present in the pIgM preparations. Twelve of 16 mIgMs hydrolyzed pre-aggregated ¹²⁵I-TTR (Table 2). The hydrolytic rates were highly variable (undetectable to 2.98nM/µg IgM/h), consistent with the prediction of differing catalysis by individual catabodies with structurally distinct V-domains. Like the pIgMs, the 12 misTTR-hydrolyzing mIgMs hydrolyzed misTTR but not phyTTR, shown by: (a) the depleted 1mer band of pre-aggregated ¹²⁵I-TTR but not non-aggregated ¹²⁵I-TTR in boiled reaction mixtures containing; and (b) the depleted 1mer TTR band originating from misTTR and the undepleted 4mer phyTTR band in non-boiled reaction mixtures of pre-aggregated ¹²⁵I-TTR (Fig. 5D,5E show the example of mIgM 1802). Moreover, the mIgMs generated the same 13, 10 and 7 kDa TTR product fragments observed for pIgMs (Inset, Fig. 5C). The consistent selectivity and product fragment profiles suggest a shared structural basis for mIgM and pIgM catalysis.

From mutagenesis [30], epitope mapping [87] and protease inhibitor studies[88], the split-site catalysis model was proposed (Fig. 6A), involving discrimination between structurally distinct antigen epitopes by the noncovalent binding site prior to peptide bond hydrolysis at the catalytic subsite. At saturating concentrations, the short peptide EAR-AMC substrate is hydrolyzed detectably by the catalytic subsite, a test for catalysis occurring without selective epitope recognition[37, 52].As reported [55], titration of mIgM Yvo with the E-hapten 1 inhibitor indicated 10.2 EAR-AMC hydrolytic sites/IgM molecule (inset, Fig. 6B; theoretical number of catalytic sites/IgM, 10). When tested as an alternate substrate, EAR-AMC inhibited the misTTR hydrolytic activity of mIgM Yvo (Fig. 6B). Ala-Ala-Ala-AMC (AAA-AMC) a peptide that is not hydrolyzed by the mIgM, did not inhibit the misTTR hydrolytic activity. It may be concluded that misTTR and EAR-AMC hydrolysis occur at the same mIgM catalytic site. In addition, EAR-AMC and misTTR hydrolysis by the panel of 16 mIgMs was significantly correlated (P=0.005; Fig. 6C), consistent with regulation of misTTR hydrolysis by the catalytic subsite efficiency. Importantly, the concentration of misTTR required for detection of hydrolysis was approximately 3-log orders lower than the EAR-AMC substrate, consistent with selective misTTR recognition. Certain mIgMs with low EAR-AMC hydrolytic activity hydrolyzed misTTR robustly (e.g., IgMs 1811, 1814 falling outside the regression confidence limits in Fig. 6C), suggesting accelerates hydrolysis due to selective misTTR recognition.

Are the catabodies polyreactive? The complementarity determining regions (CDRs) of some polyreactive IgMs produced without immunogen stimulation can bind epitopes with no discernible structural similarity [89], a phenomenon that may originate from CDR flexibility and the induced-fit binding mechanism. Consistent with previous reports [55, 56], the pIgMs and mIgMs did not hydrolyze non-amyloidogenic, non-superantigen proteins from eukaryotic and prokaryotic organisms (Fig. 7A; no hydrolysis of albumin, extracellular epidermal growth factor receptor fragment, ovalbumin, transferrin, collagen adhesion protein or LukS). Specific catalysis requires spatial coordination between the catabody noncovalent and catalytic subsites[56]. The lack of indiscriminate pIgM proteolysis may be explained by uncoordinated CDR-based, polyreactive binding activity and catalytic subsite activity.

The misTTR-hydrolyzing mIgM Yvo was reported to hydrolyze amyloid β 1-40 (Aβ40) and 1-42(Aβ42) peptides [55], a reactivity pattern reminiscent of antibodies that bind the β-strand conformational epitope of amyloids without dependence on the epitope sequence [90, 91]. In contrast, the misTTR hydrolyzing mIgM 1802 did not hydrolyze pre-aggregated Aβ42 (Fig. 7B-D), suggesting sequence-selective misTTR recognition. Only 2 of 9 misTTR-hydrolyzing mIgMs tested hydrolyzed Aβ40 (Fig. 7E). Certain misTTR-hydrolyzing mIgMs also hydrolyzed HIV protein gp120, a B cell superantigen [56]. Superantigens are recognized noncovalently at the framework regions (FRs) of reversibly-binding antibodies [92-94] and catabodies [95, 96], and the FRs also play a direct role in peptide bond hydrolysis [30]. The correlations between hydrolysis of misTTR, gp120 and another superantigenic protein, *Staphyloccus aureus* Protein A was studied [94]. A minority of misTTR-hydrolyzing mIgMs hydrolyzed Protein A (3/16 IgMs;Fig. 7F), and a majority hydrolyzed gp120 (13/16; Fig. 7G). There was no correlation between misTTR and gp120 or Protein A hydrolysis (P>0.05).Two misTTR-hydrolyzing mIgMs did not hydrolyze Aβ, Protein A or gp 120 (IgM 1802 and 1814). mIgMYvo was the only catabody with hydrolytic activity directed to all four amyloid/superantigen substrates (misTTR, Aβ, Protein A and gp120). misTTR hydrolysis by this mIgM was inhibited by the alternate substrate Aβ but not gp120 or Protein A(Fig. 7F), indicating that occupancy of the superantigen-interacting sites does not impede misTTR hydrolysis. The studies indicated variable mIgM reactivity profiles, including mIgMs monoreactive for TTR and mIgMs with amyloid/superantigen-directed oligoreactivity.

misTTR dissolution. Treatment of a 10-fold molar excess of pre-aggregated TTR with the hydrolytic mIgM 1802 but not non-hydrolytic IgM 1819 resulted in time-dependent reduction of turbidity to the near-basal value of non-aggregated TTR (Fig. 8A). ThT binding by pre-aggregated TTR, an indicator of β-sheet content, was also reduced to the near-basal value by the hydrolytic but not the non-hydrolytic mIgM (Fig. 8B). The observed dissolution rate is diffusion-restricted by the particulate substrate character, and the dissolution process likely proceeds through a series of hydrolytic reactions, eventually converting particulate misTTR to soluble products. The mIgMs (130 µg/mL) did not form detectable immune complexes (% soluble and particulate ¹²⁵I-misTTR binding: hydrolytic mIgM 1802,1.9±1.6 and 0.3±0.1%, respectively; non-hydrolytic mIgM 1819, 1.0±2.6 and 1.6±1.6%, respectively). As the hydrolytic mIgM 1802 has no misTTR binding activity, and but for the V domains, is structurally identical to the non-hydrolytic mIgM, the dissolution reaction must be due to misTTR digestion.

Small misTTR amounts are likely produced as normal byproducts of alternate TTR folding pathways. With advancing age, such misTTR species are thought to serve as seeds for pathological TTR amyloid formation. The findings indicate physiological production of misTTR-selective IgM catabodies are a first-line defense function against TTR amyloidosis. The IgMs hydrolyzed soluble and particulate misTTR, but not the non-pathogenic phyTTR tetramer found at micromolar concentrations in blood, indicating selectivity for an aggregation-induced misTTR neoepitope that is absent in phyTTR. The IgMs, therefore, can degrade misTTR with no danger of saturation by the more abundant phyTTR species. There was no evidence for IgM degradation of non-amyloid, non-superantigen proteins [55, 56]. Two misTTR-hydrolyzing mIgMs did not hydrolyze Aβ or microbial superantigens, and other mIgMs recognized these substrates at varying levels. Saturation of oligoreactive IgMs with irrelevant amyloids may limit the misTTR clearance capacity, but this is a threat only in patients with non-TTR amyloid disease. Likewise, superantigens accumulate only during microbial infections, and impairment of misTTR clearance is not predicted in the non-infected state. The selectivity properties, therefore, support a role for the IgMs in defense against TTR amyloidosis.

Further evidence for the beneficial IgM role includes: (a) Pre-aggregated TTR was dissolved completely by a hydrolytic mIgM; (b) misTTR was degraded at IgM concentrations ~200-fold lower than the blood IgM concentration, and virtually the entire misTTR degradative capacity of serum was attributable to IgMs; (c) the blood IgM concentration exceeds that of non-antibody proteases by several log orders, and pIgMs hydrolyzed misTTR at a rate comparable to a non-antibody protease (MMP-9); (d) the pIgM hydrolytic activity was refractory to serum inhibitors of non-antibody proteases; and (e) pIgM from different healthy adults hydrolyzed misTTR within a narrow rate range, indicating stable maintenance of the misTTR hydrolytic activity under disease-free conditions. The beneficial IgM function extends beyond clearance of blood-borne misTTR. IgMs permeate most anatomic blood-tissue barriers. Lymph from many tissues, a common surrogate for the tissue interstitial fluid, contains IgM in the mg/ml range [97-99], indicating the ability for tissue misTTR clearance.

Notwithstanding the favorable catabody properties, pathological TTR amyloid accumulates in ATTR patients, indicating that the catabody clearance capacity may be overwhelmed by independent age-associated factors that accelerate protein misfolding, e.g., the reaction of amyloidogenic proteins with advanced glycation and lipid peroxidation end-products [100, 101]. In addition one cannot exclude reduced catabody formation as a possible factor in TTR amyloidosis. Age-associated shifts in the structural and functional properties of antibody V-domains are documented [102, 103], individual mIgMs hydrolyzed misTTR at widely variable rates, and aged humans display increased susceptibility to infections [104]. A small, non-significant tendency towards reduced misTTR hydrolysis in healthy, aged humans was observed. Future studies on patients with ATTR may be performed to further assess whether immune senescence is a predisposing factor in amyloidosis. In addition, long-term follow-up of humans and mice with genetic antibody deficiencies [105, 106] may be useful to define the contribution of the immune system in defense against amyloidosis. The catalytic function may also be conceived to diminish the inflammatory consequences of antibody-antigen interactions. Reversible binding at the Fab region of conventional antibodies stimulates Fc-region mediated activation of complement and phagocytosis, exemplified by the inflammatory effects of Aβ-binding antibodies [107]. Rapid catabodies form only transient immune complexes and then release the digested antigen products, reducing the probability of harmful immune complex effects.

According to the acquired immunity paradigm, IgGs with specific binding activity directed to individual target epitopes are produced within a few weeks by the immunogen-driven maturation of antibody light and heavy chain variable domain sequences (respectively, VL and VH domains). Acquired immunity rules did not yield catabodies capable of complex peptide bond hydrolytic reactions [108]. Moreover, the rules provide no basis to anticipate the strict catabody selectivity for misTTR. As misTTR-directed IgMs are produced by healthy humans without TTR amyloidosis, they are likely produced constitutively without requirement for acquired immunity processes. Catabody synthesis can occur without immunogen-driven V domain maturation because the serine protease-like nucleophilic sites are encoded by heritable germline V region genes [29, 57-59]. A germline VL gene residue is an essential constituent of the misTTR-hydrolyzing mIgM Yvo catalytic triad (Ser110, IMGT numbering), and the 2 remaining catalytic residues are evidently acquired during V-(D)-J gene recombination, which occurs prior to contact with the immunogen (Arg112 and Glu113 at the VL-J and VHD-J junctions, respectively[109]. Furthermore, the IgMs are the first antibody class produced by B cells, whereas IgGs generated at the terminal acquired immunity stage are poorly hydrolytic. The antibody C domain scaffold can influence antigen binding at the remote V domains [110]. C domain swapping studies indicated loss of catalytic activity at the IgM→IgG class-switch step due to the unfavorable effect of the IgG C domains on the V domain catalytic site [31]. Detailed information about factors regulating IgM constitutive synthesis is not available. However, IgMs are synthesized at stable levels by germ-free animals [111], and their constitutive production may involve an autonomous B cell developmental program and BCR-independent lymphocyte growth stimulators [35].

In adult humans, IgGs are the dominant blood-borne antibodies, found at 5-6 fold greater concentrations than IgMs. Many IgGs express specific antigen binding activity, consistent with maturation of their V-domains by immunogen-driven somatic mutation process. Such antigen-specific IgGs are the basis of protective responses induced by microbes and vaccines, and numerous antigen-binding monoclonal IgGs are approved for therapeutic use [112]. Catalytic IgG preparations from patients with autoimmune disease are reported to hydrolyze self-antigens [40, 113-115], but dysfunctional B cell reactivity in autoimmune disease may contribute to increased catalytic IgG synthesis [40], and the hydrolytic rate of the IgGs is usually lower than IgMs [116, 117]. Monoclonal IgGs from routine immunization protocols display minimal or no catalytic activity, but their dissociated light subunit frequently hydrolyzes peptide bonds [118-120]. Other IgGs generated by healthy humans express low-affinity, polyreactive antigen binding profiles (often designated "natural" IgGs) that may originate from occurrence of class-switch recombination in bystander B cells without sufficient mutations in the V domains required to accomplish specific antigen binding [121, 122]. The polyreactivity was suggested to contribute in the therapeutic effect of pooled IgGs from healthy humans in patients with bacterial infections and certain autoimmune diseases (commonly known as intravenous immune globulin or IVIG preparations) [123]. The catalytic activity of such IVIG preparations is marginal compared to IgMs [74]. Also, the IgMs in the present study display catalytic specificities that are readily distinguishable from the polyreactive IgG binding pattern.

Protein-protein recognition is dictated by sequence-dependent chemical interactions, topographical shape complementarity and local flexibility of the reacting sites. The amyloid-superantigen link is obvious from mIgMs with oligoreactivity for both protein classes, e.g., mIgM Yvo. Comparison of linear sequences did not indicate a homologous epitope that is shared by TTR, Aβ, Protein A and gp120 (Fig. 9A). The innate catabody model conceives an initial selectivity-conferring binding step that places the substrate peptide backbone in spatial register with the catabody hydrolytic subsite [30, 40]. The oligoreactivity may originate from substrate interactions at a single cross-reactive catabody site or multiple catabody sites with unique ligand preferences. Antibody V domains are described to express multiple ligand-interacting sites outside the classical CDR-based antigen binding pocket [124]. For instance, the FR-based VH domain sites that recognize Protein A and gp120are not identical [92, 93], and yet another VL domain site recognizes the superantigen Protein L[125]. As misTTR hydrolysis by mIgM Yvo was not inhibited competitively by Protein A and gp120, non-identical sites are likely involved in hydrolysis of misTTR versus superantigens. In contrast, Aβ inhibited the hydrolysis of misTTR, indicating amyloid substrate recognition at a shared IgM site. A precedent for the TTR/Aβ cross-reactive site is available - reversibly-binding antibodies can recognize the β-strand conformational epitope found in amyloid proteins with differing sequence [90, 91].

Humans produce no more than 100,000 non-antibody proteins, including all known enzymes and receptors with specificity for a broad range of ligands. The recombined IgM repertoire derived from the germline V, D and J segment configurations is far larger (from germline gene sequences available in IgBLAST, ~4x10⁹ VL-VH domain pairs derived by recombination of 152 VL with 19 J genes and recombination of 273 VH genes with 34 D and 13 J genes; this estimate excludes further repertoire expansion occurring during the junctional diversification process). The human germline IgM repertoire is a source of catabodies with functionally useful selectivity for individual substrates likely developed by Darwinian immune evolution (Fig. 9B). Like the misTTR-selective catabodies, the innate character of catabodies to Aβ is evident from superior Aβ hydrolysis by IgMs compared to IgGs and by production of Aβ binding and hydrolyzing antibodies by healthy humans without amyloidosis [55, 126]. Jawed fish, the first extant organisms with an immune system orthologous to humans, produce TTR and Aβ with sequences similar to their human counterparts (Fig. 9C). As tissue amyloid deposition has no known benefit in eukaryotes, the catabodies may have evolved to retard amyloidosis. Amyloids of 27 proteins can accumulate to pathogenic levels, causing premature senescence and disease [1]. Proteins accumulate in the amyloid state within minutes to days in vitro, indicating the existence of functionally important mechanisms that maintain amyloids at non-toxic levels in vivo. Amyloidosis occurring prior to reproduction will jeopardize survival of the species. Increased misfolded protein levels can be detected years prior to appearance of disease symptoms, e.g., toxic Aβ oligomers in middle-aged humans [127] and particulate Aβ deposits in reproducing monkeys [128].Moreover, young humans are susceptible to amyloidosis in conjunction with cardiovascular disease, infections, cancer and exposure to environmental toxins[129-131], e.g., atrial natriuretic peptide amyloidosis in congestive heart failure [132]. Thus, there is a role of innate amyloid-clearing catabodies in furnishing an evolutionary survival advantage. Catabodies to the superantigens HIV gp120 [56, 133], S. *aureus* Efb [134] and S. *aureus* Protein A serve as innate defense mediators against microbes. Superantigens, however, downregulate acquired B- and T-cell immunity, and the survival advantage for the microbe gained by evading host acquired immunity [94] may trump the protective function of anti-superantigen catabodies.

In summary, the physiological production of misTTR-selective catabodies has been shown. Unlike other approaches to suppression of TTR amyloidosis, the catabodies remove misTTR without interfering in the essential functions of the properly-folded TTR state. The misTTR-hydrolyzing IgMs, therefore, hold potential for developing a safe treatment for TTR amyloidosis. Several monoclonal IgMs have been approved in the USA for human tests, and pentamer IgM-containing polyclonal antibodies are approved for human therapy in Europe [135]. Converting pentamer IgM to the monomer IgM state does not compromise the catalytic activity [37], and monomer IgMs can be considered as effective therapeutic reagents. Small amounts of IgM monomers are found physiologically in humans [136], and lower organisms produce larger monomer IgM amounts [54]. In addition, highly catalytic single VL domains and paired VL domains can be engineered [33, 40] as candidate drugs from the misTTR-hydrolyzing IgMs.

### EXAMPLE 2: ISOLATION OF RENEWABLE CATABODY SOURCES WITH misTTR-DIRECTED AND POLYREACTIVE CATALYTIC ACTIVITY

The misTTR-selective catabodies fulfill a homeostatic surveillance function for removing small misTTR amounts formed as the byproduct of the TTR folding pathways. The catabodies can also be applied for removing tissue amyloid deposits. Renewable monoclonal catabody sources prepared from the B cells of healthy humans are described. The catabody properties pertinent to the anti-amyloid defense function are also described.

### Renewable catabody sources

Nucleophilic catalysis is an innate, germline V-gene encoded function. For TTR amyloid, the noncovalent specificity-conferring function is also an innate catabody property [5]. The catalytic activity is best expressed by IgMs produced early in B cell ontogeny [37, 137].The covalent reactivity of electrophilic phosphonate probes with nucleophilic IgM class BCRs was documented [37]. To select specific monoclonal catabodies, electrophilic E-misTTR that binds B cells from healthy humans specifically was used (see Example 1). This reagent identifies IgM class BCRs that recognize the unique misTTR epitope in coordination with nucleophilic attack on peptide bonds. Following transformation with Epstein-Barr virus (EBV), a screen of the top 30% E-misTTR labeled B cells identified secreted monoclonal IgMs with misTTR-hydrolyzing rates >50-fold superior to the reported rate of IgMs from patients with Waldenström's macroglobulinemia (Fig. 10A-C).The E-misTTR labeled B cells were recovered by flow cytrometric sorting and plated at limiting dilution with irradiated peripheral blood mononuclear cells serving as feeder cells. The cells were immortalized by treatment with the supernatant of the EBV-infected cell line B95-8 and IgM secretion was monitored by ELISA 3 weeks thereafter. The transformation efficiency was -20%. Cell lines secreting IgM at >250 ng/mL were analyzed for catalytic activity. No phyTTR hydrolysis was detected. From hydrolysis assay performed with unfractionated tissue culture supernatant, the following monoclonal IgM clones with misTTR hydrolytic activity were identified using E-misTTR-selected B cells as the starting materials: IgM clones 3D12, 10E10, 6H1 and 9D5.The catalytic activity was verified using the purified IgMs. Additional IgM clones with misTTR hydrolytic activity identified by screening of purified IgMs are: unselected IgM clone 4G2 and E-misTTR-selected IgM clones 9E11, 8H6, 8D5, 9F3 and 7D11.

To isolate catabodies with the highest possible rate, only the top 1% of E-misTTR labeled B cells are transformed with EBV, ensuring identification of the IgM BCRs with greatest specificity and catalytic activity directed against misTTR. The method consists of preparing B cells expressing IgM class BCRs by routine magnetic bead kits or flow cytometric sorting from human peripheral blood, followed by treatment of cells with the soluble E-misTTR probe (obtained as the supernatant from a mixture of particulate and soluble E-misTTR [5]). Noncovalently bound probe is removed by extensive washing or incubation of the cells with excess misTTR or hapten 1 devoid of the electrophilic group. The covalently bound B cell fraction is transformed with EBV. Secreted IgMs from 20 or more wells containing the cultures cells are screened for ability to hydrolyze ¹²⁵I-misTTR, ¹²⁵I-phyTTR (by electrophoresis) and Glu-Ala-Arg-AMC (by fluorimetry). Glu-Ala-Arg-AMC hydrolysis measures overall catalytic efficiency independent of epitope recognition. Other small dipeptides, tripeptides and tetrapeptides devoid of an antigenic epitope can also be used to identify polyreactive hydrolytic activity of the antibodies. For the purpose of the instant disclosure, an antigenic epitope is defined as a peptide epitope composed at a minimum of 5 amino acids. Maintenance of IgM catalytic activity following removal of any trace contaminants by denaturing chromatography is documented [37]. Catalysis assay errors are <15%. The U- and t-tests are used to determine the significance of differences in catalytic activity. Stable cell lines are obtained by cloning the VL/VH cDNA (obtained by the reverse transcriptase-PCR method) adjacent to human κ and µ C-domains (pLC-huCK, pHC-huCµ vectors) and expressed in NS0 cells as described [96, 138].

The same procedure is applied to isolate polyreactive catabody-secreting B cells, except that the initial B cell selection is conducted with the E-hapten 1 probe containing the electrophilic phosphonate but not an antigenic epitope (Fig. 10D). As a result, the procedure selects for epitope-independent B cells secreting efficient polyreactive catabodies. Improved reactivity of the B cells with E-hapten 1 is verified flow cytometrically and microscopically by comparison with hapten 1 containing the minimally electrophilic phosphonic acid group (instead of the highly electrophilic esterified phosphonate group) [5, 37]. Following transformation with EBV, the identification of B cell lines that secrete high efficiency polyreactive catabodies into the supernatants is confirmed by screening using the Glu-Ala-Arg-AMC or other protein substrates.

Alternate cellular sources and methods to isolate the desired catabodies are available. For example, IgMs from umbilical cord blood B cells with no or minimally mutated V-genes may express high level catabody activity, as they represent the naive human antibody repertoire that is minimally biased by memory B cell responses to external immunogens. Conversely, adaptive immunity effects such exposure to naturally-occurring electrophilic analogs of misTTR (NE-misTTR) and other antigens (NE-antigens) may result in amplification of catabody activity in old age or during autoimmune disease processes. Thus, high activity catabodies, including the polyreactive catabodies could be obtained from the cells of old humans, patients with amyloidosis or patient with autoimmune disease. Instead of EBV transformation, the VL and VH cDNA can be rescued from single B cells following selection with the E-misTTR, E-hapten 1 or NE-antigens by the reverse transcriptase-polymerase chain reaction[139], cloned adjacent to human κ and µ C-domains and expressed in a suitable host cell line[31, 140].

In addition to intact catabodies, the unpaired human light chains and unpaired shark or camel single V-domains can be isolated by phage display methods as renewable catabody sources. Unpaired human light chains hydrolyze small peptides rapidly compared to paired IgG VL-VH domains[32]. Jawed fish produce stable single V-domains attached to a heavy chain C-domain. Human light chain and shark single V-domain libraries are available [141, 142].The libraries displayed on phages and phage selection are done by covalent binding to E-misTTR or E-hapten 1[33].

In addition, high activity catabodies can be obtained by B cell or phage display selection procedures conducted using the NE-antigens, including NE-misTTR. NE-antigens containing aldehydes and ketones are formed by the reaction of the protein epitopes with advanced glycation/lipid peroxidation end-products (AGEs, LPEs; Fig11). These compounds can react covalently with nucleophilic BCRs and stimulate catabody synthesis. Studies on catabodies to Aβ and the electrophilic Aβ-malondialdehyde adduct have validated covalent binding of the natural electrophile to the catabody nucleophilic groups (Fig12A-C). As examples, NE-misTTR1-5 derivatives with electrophiles at Lys, Arg, His, and Cys side chains by treating pre-aggregated misTTR with these AGEs/LPEs: 4-hydroxy-2-nonenal, malondialdehyde, methylglyoxal, glyceraldehyde, glyceraldehyde-3-phosphate [101]. The reaction is monitored by electrophoresis and staining with a small-molecule nucleophile (biotin-hydrazide). Oligomer/fibril formation is monitored by electrophoresis, gel filtration, turbidometry and ThT fluorimetry. Covalent binding of NE-antigens to catabodies and BCRs is confirmed by measuring NE-antigen release from immune complexes in a non-denaturing solvent [38]. For further validation, the covalent binding rate (k3), noncovalent epitope binding (1/KD) and irreversible NE-antigen inhibition of substrate hydrolysis by the catabodies can be measured [143].

### Catabody characterization

Catabodies monoreactive for misTTR can be characterized by standard immunochemistry, molecular biology and enzymology methods. In addition, the novel specificity of properties of polyreactive catabodies pertinent to disease treatment and diagnosis can be revealed from these tests.

Innate origin and valency effects. Unfractionated culture supernatants and IgMs purified on immobilized anti-human IgM will be tested. The misTTR hydrolytic activity of culture supernatants from the B cell-lines is almost exclusively due to secreted IgMs, with little or no background activity due to cellular proteases. Catabodies from E-misTTR selected cell-lines display superior misTTR hydrolytic activity but not superior Glu-Ala-Arg-AMC hydrolytic activity compared to the E-hapten 1 selected cell-lines. Conversely, polyreactive catabodies from E-hapten 1 selected cell-lines should display superior Glu-Ala-Arg-AMC hydrolytic activity compared to the E-misTTR selected cell lines. The VL/VH domain cDNA of the catabodies and the N-termini of the V-domain proteins is sequenced. Unmutated V-genes will suggest germline-encoded catalytic activity. Replacement/silent mutation ratios that do not exceed the random mutational ratio suggest catabody production without immunogen-driven selection pressures. Over-representation of individual germline VL/VH genes in the panel identifies V-gene(s) encoding innate misTTR specificity. Expression of catalysis by pentamer IgM is confirmed by gel filtration. Monomer IgM is detectable in human serum [136]. The smaller monomers may be the preferred species for potential clinical applications. Monomer IgMs are produced by mild reduction and akylation of the pentamer or expression in an appropriate cell cell line that minimizes oligomerization of the monomers. There was no loss of Glu-Ala-Arg-AMC hydrolytic activity upon pentamer IgM conversion to monomer IgM [31]. Avidity effects due to multivalency are not a factor in solution state assays for substrates such as Glu-Ala-Arg-AMC that are devoid of repeat epitopes but may contribute to the rate for TTR aggregates. misTTR hydrolytic activity of enzymatically-prepared monovalent IgM Fab fragments can also be tested [37]. The IgM C-domains scaffold exerts a favorable effect on the V-domain catalytic site [31]. To explain the failure of IgGs to hydrolyze misTTR[5], the V- domains of an IgMs can be cloned as an IgG, and the hydrolytic activity of the V-domain identical IgM and IgG is compared.

Specificity. Catabody specificity can be determined as follows: (a) Substrate specificity using wildtype and mutant misTTR species; amyloids formed by proteins unrelated in sequence to TTR; microbial superantigens with known sensitivity to catabodies; non-oxidized extracellular proteins free of known amyloid or superantigen character; oxidized amyloid, oxidized superantigens, and oxidized extracellular proteins free of amyloid or superantigen character; non-oxidized and oxidized intracellular proteins; and (b) Scissile bonds and noncovalent binding epitope; and (c) misTTR amyloid dissolution.

Substrate degradation can be monitored by electrophoresis, gel filtration, HPLC or mass spectroscopy. If required a radioactive label, biotin or a fluorescent label is inserted into the substrate, and immunoblotting with antibodies to various substrate epitopes is applied to visualize depletion of the intact protein and appearance of fragments is analyzed as in previously published hydrolysis assays. Specificity can be tested using: (a) Separately, the soluble and fibrillar misTTR species prepared from wildtype TTR and mutant TTR substrates (e.g., V122I, V30M, L55P, D18G and A25T mutants). The mutants aggregate at varying rates and cause hereditary amyloidosis with differing disease phenotype (neuropathy cardiomyopathy[144] (b) Non-TTR amyloids prepared from human Aβ42 peptide[145], a κ-light chain from a multiple myeloma patient[146], α-synuclein[46], atrial natriuretic peptide, islet amyloid polypeptide, and tau[147]; (c) The superantigen proteins HIV gp120 and *Staphylococcus* Protein A[5]; (d) a panel of 10 or more extracellular proteins without amyloid or superantigen character[5];(e) the oxidized versions of the aforenamed amyloid proteins, superantigens and extracellular proteins prepared by the reactions with AGEs and LPEs as specified under the section "renewable catabody sources"; and (f) an extract of intracellular antigens obtained by disruption of human cells (e.g., by detergent extraction of neurons or myocytes) with and without prior oxidation with the AGEs and LPEs.

The misTTR epitope recognized noncovalently by catabodies is likely the major determinant of specificity, and individual amino acids at the scissile bonds may also be a contributory factor. Time course studies can reveal whether the initial misTTR products of the catalytic reaction are digested further into smaller products. Multiple cleavage sites suggest the split-site catabody model, in which the catalytic subsite is in register with alternate, spatially-neighboring peptide bonds following initial noncovalent binding [40, 87]. The electrophilic antigen analog binds covalently and specifically to the two catabody subsites (Fig 13). Reuse of initial products as substrates is feasible provided the noncovalent binding epitope is intact. The scissile bonds is identified by matrix-assisted laser desorption/ionization-time of flight mass spectroscopy of reaction mixtures and N-terminal sequencing[33, 37]. The epitope may be located distant from the scissile bonds. Residues 41-50 and 115-124 hold potential as unique epitopes expressed by misTTR but not phyTTR, as suggested by studies on TTR-binding antibodies [148-150]. There is no evidence for a conformational (discontinuous) misTTR epitope that is not expressed by phyTTR. Overlapping 20-mer synthetic TTR peptides are tested as inhibitors of misTTR hydrolysis. To confirm epitope identity, full-length mutant TTR containing Ala replacements at residues within the candidate amyloid epitope is tested as substrate. Reduced catalysis suggests specific epitope recognition.

Essentially the same procedures can be applied to identify the scissile bonds and epitope recognized by the catabodies in other substrate proteins found to be sensitive to catabody hydrolytic activity.

Efficient catabodies do not form stable immune complexes. This can be confirmed by tests of catabody binding to the substrates by routine ELISA and surface plasmon resonance methods

Active site. Conventional antigens bind antibody CDRs. The V-domain framework regions (FRs) may have developed amyloid recognition capability early in immune evolution because of the potential survival advantage of an anti-amyloid antibody function [5]. The FRs are well known to contribute amino acids essential for catalysis [30, 57, 58] and noncovalent binding sites for B cell superantigens [92, 93]. The role of the FRs may be confirmed by swapping the FRs of a catabody (e.g., a misTTR-directed catabody with the corresponding FRs of an irrelevant antibody (one FR at-a-time; 8 mutants) using the IgM monomer scaffold, which supports efficient catalysis. This approach has been applied to localize superantigen binding sites [96]. Catalytic rates are measured. A change of Km and maximal velocity (Vmax) indicates, respectively, disruption of the noncovalent binding site and catalytic site. Noncovalent binding cavities and nucleophilic sites may be deduced from V-domain molecular models [95]. The catalytic nucleophile may be identified from the site of the covalent reaction with the E-hapten-1, a validated method for identifying enzymatic nucleophiles by enzymatic digestion and mass spectroscopy [143, 151]. Together, the studies reveal the V-domain residues important for catalysis and noncovalent substrate binding.

misTTR dissolution. Treatment with the E-misTTR-selected monoclonal IgM clone 10E10 dissolved particulate misTTR (Fig14). Dissolution of particulate misTTR by the IgMs may be measured at varying IgM concentrations by the turbidometry and the ThT fluorimetry methods (see Example 1). Dissolution of particulate mutant misTTR may be measured similarly (e.g., V122I, V30M, L55P, D18G and A25T). Monoclonal IgMs with misTTR-specificity, catalytic activity and dissolution capability will be effective therapeutics.

### EXAMPLE 3: ADAPTIVE AMPLIFICATION OF NUCLEOPHILIC CATABODIES BY misTTR AND ELECTROPHILIC misTTR ANALOGS

Non-electrophilic immunogens do not generally induce catabodies as B cell differentiation is driven by immunogen binding, and catalytic immunogen hydrolysis is not known to be a selection pressure for B cell growth and development into antibody-secreting cells [40, 152]. In contrast, as the misTTR-directed catalytic activity is an innate property, the E-misTTR and NE-misTTR analogs described in the Examples 1 and 2 induce adaptive strengthening of the innate BCR nucleophilicity coordinated with improved noncovalent binding of misTTR. This is supported by previous studies that electrophilic analogs of gp120 induce the synthesis of specific catabodies to gp120 [39]. Application of the E-misTTR and NE-misTTR immunogens is useful for isolating therapeutic catabodies to misTTR. In addition, these immunogens are useful to develop prophylactic and therapeutic vaccines against amyloidosis, both ATTRwt and ATTRm.

### E-misTTR and NE-misTTR immunogens

The immunogens may be prepared by the incorporation of electrophilic phosphonate groups or carbonyl groups of the AGEs and LPEs (Fig12A) into misTTR. The reaction may be monitored by electrophoresis and staining with a small-molecule nucleophile (biotin-hydrazide). Aggregation of the misTTR may be monitored by electrophoresis, turbidometry and ThT fluorimetry, formation of covalent adducts of the E-misTTR or NE-misTTR with reference nucleophilic antibodies may be monitored by denaturing electrophoresis.

### Induction of catabody synthesis

Catabody synthesis may be accomplished, for example, in groups of 6 mice that receive 4 subcutaneous immunogen administrations at 4 wk intervals. A non-toxic adjuvant (alum/RIBI) may be included to recruit antigen-presenting and T helper (TH) cells. Induction of antibodies may be monitored at 2 week intervals in blood by ELISA using immunogen-coated plates. Antibodies with reversible and covalent binding activity for the immunogen may be distinguished in ELISA plates without and with SDS treatment [39]. IgM and IgG antibodies may be purified from high-titer sera (>1:5000) and covalent binding may be confirmed by electrophoresis. Then, the antibodies may be tested for cleavage of misTTR and physiological TTR tetramers as in Example 1. It is desirable to establish the following properties of polyclonal antibodies: (a) Catalytic specificity using various amyloids and irrelevant proteins; (b) Scissile bonds of wildtype misTTR and hydrolysis rates of mutant misTTRs; (c) Noncovalent binding epitope; and (d) Dissolution of preformed aggregates of wildtype and mutant misTTR preparations.

### Monoclonal catabodies

Individual monoclonal antibodies generally display greater target antigen recognition compared to polyclonal antibody mixtures. Monoclonal catabodies may be prepared using standard hybridoma technology from mice immunized with the E-misTTTR or NE-misTTR immunogens [39]. Monoclonal IgMs/IgGs with immunogen binding activity may be tested for hydrolysis of misTTR as in Example 1, and the catabodies may be characterized for specificity, hydrolytic rate and TTR amyloid dissolution rate as in Examples 1 and 2.

### EXAMPLE 4: CLEARANCE OF MISTTR AND MITIGATION OF MISTTR TOXICITY BY CATABODIES

No pharmacologically effective treatment is available for ATTRwt or ATTRm. Dissolution and detoxification of misTTR may be tested using test tube methods. Clearance of misTTR may also be tested in transgenic mice expressing human TTR infused with catabodies.

### Anti-amyloid effects in vitro

Examples 1 and 2 describe tests of dissolution of preformed wildtype misTTR and mutant misTTR aggregates (V122I, V30M, L55P, D18G and A25T) by the catabodies. Additional in vitro studies may be done to define the activity profile of the best catabodies with the desired specificity and catalytic properties. Both the physiological pentamer IgM form and the smaller monomer IgM form capable of penetrating various tissues at higher levels may be tested (obtained, respectively from expression in J-chain positive NS0 cells and J-chain negative CHO cells). In addition to verifying dissolution of preformed TTR amyloid, inhibition of formation of wildtype and mutant misTTR aggregates may be measured in the presence of the IgMs by turbiditometry/ThT fluorimetry. The data may be expressed as the IgM concentrations required to achieve 50% inhibition of misTTR amyloids. Dissolution of TTR amyloid may be confirmed by transmission electron microscopy after adsorption to copper grids and staining with uranyl acetate. To confirm recognition of "native" amyloid, catabody dissolution of amyloid deposits in cardiac tissue from ATTRwt patients and ATTRm (V122I, V30M) may be tested. Tissue cryostat sections may be treated with test and control IgMs, followed by staining with Congo red or available monoclonal IgGs that bind TTR specifically and estimation of amyloid deposits by microscopy. The tissue deposits may contain TTR fragments trapped within the amyloid formed by full-length misTTR [153, 154]. The dissolution of the cardiac amyloid deposits found in early-onset ATTRm as well as late-onset ATTRm containing higher and lower full-length protein/fragment ratios, respectively [154], may be tested. Mutant TTR amyloid is known to contain wildtype TTR [153] that is sensitive to the catabodies.

Soluble oligomers of amyloidogenic proteins are toxic [11]. Soluble wildtype and mutant misTTR oligomers may be separated from fibrillar TTR by centrifugation. The toxicity of the soluble misTTR oligomers may be tested using the human myocyte cell line AC16 [12] in the presence of varying test and control IgM concentrations. Cell death may be measured by the resazurin dye reduction assay.

### Amyloid removal by catabodies

Tg mouse models expressing human wildtype TTR and V30M mutant TTR may be applied as models for testing infused catabodies. Tissue TTR deposits in the transgenic mice expressing wildtype human TTR (Tg-TTR mice) are evident at age 20 months in the kidneys and heart of -90% mice [155]. TTR deposition in the Tg-V30MTTR mouse strain in the gut is evident at 11 months and spreads to the heart, lung and intestine at 14 months [156]. Although TTR deposition in both Tg mouse strains is less robust compared to human ATTRwt and ATTRm, these mice are the only *in vivo* resource for testing drug effects[157].

The following catabodies may be tested in groups of 12 mice each: (a) A misTTR-hydrolyzing pentamer IgM; (b) A misTTR-hydrolyzing monomer IgM; (c) and (d), respectively, a control pentamer IgM and a control monomer IgM devoid of misTTR-hydrolyzing or binding activity (statistical power at P<0.05 and effect size 1.2, 80%). Chimeric antibodies containing the human V domains linked to the murine IgM C-domains may be constructed per published methods [31]. This is necessary to avoid a murine anti-human IgM response in the mouse hosts receiving the test antibodies. The draining lymph from various tissues, a surrogate of the tissue interstitial fluid, contains pentamer IgM at 30-40% of blood IgM concentrations [97, 98]. Several FDA-approved chimeric IgGs containing murine V domains failed to induce an unacceptable anti-V domain response in humans. IgMs were approved for human clinical trials by the FDA [158].

Two types of studies may be conducted. First, aged Tg mice with pre-existing tissue TTR deposits may receive 2 intraperitoneal injections of the test and control catabodies at 2 week intervals (wildtypeTg-TTR mice, 20 months age; mutant Tg-V30MTTR mice, age 14 months). This protocol assesses the catabody therapeutic potential. Second, the catabodies may be injected at monthly intervals from age 1 month through 20 months (wildtypeTg-TTR mice) or 1 month through 14 months (mutant Tg-V30M TTR mice). In both studies, tissue TTR deposits may be visualized by quantitative immunochemistry 7 days after the final antibody injection (sections of kidney, esophagus, stomach and heart). The magnitude of TTR deposition and the number of mice positive for TTR deposits may be scored on an organ-by-organ basis. Total TTR in tissue extracts and blood may be measured [148]. Inflammatory effects may be evaluated by staining for markers of activated macrophages, complement activation, T cells and B cells. At 12 mice/group, *in vivo* studies are powered to detect an effect size of 1.2 (80% power).

The half-life and tissue biodistribution of the ¹²⁵I-labeled catabody injected intravenously in mice may be determined by measuring the radioactivity in blood and various tissues (1 h-14 days)[159]. Catabody molecular integrity in samples may be monitored by electrophoresis. Pentamer IgM half-life in blood is 2-3 days [160].

### EXAMPLE 5: CATABODY DISEASE ASSOCIATION AND DISEASE DIAGNOSIS

The "carbonyl stress state" characteristic of inflammation results in formation of oxidized misTTR with enhanced amyloid-forming tendency [13]. Accumulation of toxic oligomers of amyloidogenic proteins and amyloid deposits is detected decades prior to evidence of disease [7-9]. When misTTR formation exceeds the catabody clearance rate, amyloidosis will occur (Fig 15).

Catabodies are produced innately by B cells. Basal and misTTR-induced catabody production is subject to various constitutive (immunogen-independent) and immunogen-induced B cell regulatory processes. The blood of healthy humans subjects <35 years age display variable levels of catabodies directed to various target antigens (Fig. 4A), and reduced production of catabodies to misTTR may be a predisposing factor for subsequent appearance of clinically symptomatic ATTRwt and ATTRm (Fig 15).

misTTR can act as an immunogen for B cells. Immunogens induce biphasic effects on B cells. Small immunogen amounts enhance antibody synthesis, whereas excess immunogen is a toleragen that saturates the BCR, driving the cells into apoptosis or anergy [161, 162]. Exposure to excess misTTR in ATTRwt patients, therefore, may tolerize B cells. misTTR itself or its oxidized variants with electrophilic character may act as toleragens (the NE-misTTR in Example 2). The NE-misTTR is likely to react covalently with nucleophilic BCRs. As the covalent reaction is irreversible, NE-misTTR derivatives will saturate the BCRs efficiently, causing a left-ward shift in the B cell dose response curve compared to a conventional non-covalently-binding immunogen (Fig 16). Saturation of the misTTR-selective BCRs is predicted at concentrations of E-misTTR that result only in partial occupancy of the polyreactive BCRs - this follows from the superior reaction of E-misTTR with misTTR-selective B cells facilitated by noncovalent epitope recognition. Consequently, NE-misTTR may tolerize the misTTR-selective cells but stimulate the production of polyreactive catabodies.

### Human catabody disease-association

The sera of ATTRwt patients displayed reduced catabody activity directed against misTTR, indicating impaired defense against TTR amyloid. Epidemiological details of the ATTRwt patients obtained from the Mayo clinic (provided by Drs. Robert Kyle and Angela Dispenzieri) and the two cohorts of control age-matched healthy humans from the Univ of Texas are provided in Table 4. The reduction of misTTR hydrolysis was reproducibly observed in tests of the purified IgM preparations from ATTRwt patients (Fig 17A). No hydrolysis of the phyTTR form by the IgM preparations was evident. The same IgM preparations from ATTRwt patients displayed significantly increased polyreactive catabody activity determined using the Glu-Ala-Arg-AMC substrate (Fig 17B), indicating that the polyreactive catabody subset is a useful secondary biomarker for TTR amyloidosis (Table 5). Essentially all of the misTTR hydrolytic activity of unfractionated serum from healthy humans was attributable to IgMs [5]. The reduction of misTTR hydrolytic activity observed using purified IgMs from ATTRwt patients was also evident in tests of unfractionated sera from the same patients and a control healthy human cohort (Fig 18).

**Table 4.Epidemiological details of the ATTRwt patients and control age-matched healthy humans.**

| Study group details | Healthy (cohort 1) | Healthy (cohort 2) | ATTRwt |
|---|---|---|---|
| N | 18 | 18 | 25 |
| N, female | 6 | 8 | 5 |
| N, male | 12 | 10 | 20 |
| Age range, years | 54-81 | 69-85 | 60-94 |
| Age mean, years | 71.3±7.6 | 77.1±4.6 | 79.7±6.6 |

**Table 5. Accuracy of individual misTTR hydrolysis assay, individual polyreactive hydrolysis assay and combined misTTR hydrolysis and polyreactive hydrolysis assays (middle column). Indicated parameters computed from data in Fig 17A and 17B.**

| **Parameter** | **misTTR** | **misTTR + EAR-AMC** | **EAR-AMC** |
|---|---|---|---|
| Sensitivity | 84% | 96% | 76% |
| Specificity | 61% | 94% | 65% |
| Precision | 75% | 96% | 76% |
| Negative Predictive value | 73% | 94% | 65% |
| False positive rate (α) | 0.39 | 0.06 | 0.35 |
| False positive rate (β) | 0.16 | 0.04 | 0.24 |

Currently, there is no established non-invasive and cost-effective procedure for diagnosis of ATTRwt, and tissue biopsy followed by immunohistochemical detection of TTR amyloid is the only definitive diagnostic method. The prognostic and diagnostic utility of catabodies may be established from analysis of their relationship with age in healthy humans and the occurrence of amyloidosis in patients with a diagnosis of ATTRwt or ATTRm obtained from the biopsy procedures. In addition, it is important to monitor disease severity in individual patients so that their treatment can be tailored appropriately.

Hydrolysis of misTTR and Glu-Ala-Arg-AMC by purified IgM preparations, purified IgG preparations and unfractionated sera may be analyzed. The following groups of subjects may be studied:
Normal healthy humans. Subjects without evidence of amyloid disease or infection belonging to these age groups: (i) 18-30y; (ii) 30-50y; (iii) 50-70y; and (iv) 70-90y. As an example, subgroups i-iii are composed of 12 subjects each. Subgroup iv is composed of 25 subjects (the larger n value enables valid comparison with ATTR patients below). Catabody activity may be plotted versus age. Comparison between the young 18-35y and old >70-90y subgroups provides information about changes in the catabody activity towards the end of the human life span (at P<0.05 and effect size of 1.0, power 80%). As males are more predisposed to ATTRwt compared to females, the catabody associations with gender may be assessed. For valid comparison with the V122I ATTRm patients described below, another subgroup of 25 healthy African-Americans (age 50-80 years) may be included.

Wildtype TTR amyloidosis patients with cardiac myopathy (ATTRwt patients). In addition to the patient cohort reported in Fig 17 (n=25), a second independent ATTRwt cohort may be analyzed (n=25, generally >60 years age). Amyloidosis may be diagnosed by staining biopsied cardiac tissue with Congo red and identifying TTR in the deposits by immunochemical or mass spectrometric analyses. Amyloidotic cardiomyopathy is defined as interventricular septal wall thickness (IVST)> 12 mm on echocardiogram and brain natriuretic peptide (BNP)>100 pg/mL. Neuropathy is determined from the NIS+7 score, which combines clinical assessment of muscle weakness, sensory loss and decreased stretch reflexes with several nerve conduction test attributes[163].TTR mutations may be excluded by TTR gene sequencing and mass spectrometry[164]. Catabody activities in the ATTR group may be compared with age- and gender-matched healthy humans from the preceding paragraph (at P<0.05 and effect size of 0.8, power 80%). Correlations between catabodies and the severity of cardiomyopathy (IVST, BNP), neuropathy (NIS+7 score) or survival may be determined. Various uncontrolled factors in different patients may influence catabody levels. Therefore, the catabodies may also be measured in longitudinally-collected blood samples at 12 month intervals in 10 patients (4 or more samples/patient). The catabody levels in 10 ATTRwt patients without cardiomyopathy but with biopsy-confirmed TTR amyloidosis in extra-cardiac sites may also be measured.

Hereditary mutant TTR amyloidosis patients (ATTRm patients). Patients expressing the V122I TTR mutation found in 3-4% African Americans with frequent cardiomyopathy and V30M TTR mutation in Caucasians with frequent polyneuropathy may be studied (n=25 each patients each). The mutations may be determined from the TTR gene sequence and mass-spectrometery in biopsied tissue [164]. Catabody hydrolysis of the Y78:F mutant misTTR responsible for hereditary ATTRm has been verified. The V122I and V30M single mutations are also unlikely to markedly alter the cleavage susceptibility of mutant misTTR species to catabodies. Catabody activities may be compared with age- and gender-matched healthy humans (at P<0.05 and effect size of 0.8, power 80%). The V122I misTTR and V30M misTTR substrates may be tested. The rates of mutant TTR and wildtype TTR by the catabodies may be compared.

All samples from the test human subjects are also analyzed for binding of misTTR and phyTTR. If binding is observed, the correlation with hydrolytic activity may be analyzed. Interference in the assay by blood-borne misTTR is unlikely, as such species are difficult to detect in the blood of ATTRwt patients by routine binding assays [65].

Observations of suppressed misTTR-directed IgM catabodies accompanied by increased Glu-Ala-Arg-AMC cleaving polyreactive catabodies indicate a blood- based ATTRwt diagnostic test. The assay sensitivity, specificity, precision, false positive rate and false negative rate may be computed for the individual and combined assay data as in Table 5. Correlation of the catabody activities with the severity of cardiomyopathy or neuropathy indicates the ability to monitor disease progression.

To rule out non-specific factors, misTTR and Glu-Ala-Arg-AMC hydrolysis by IgM catabodies from control patients with an autoimmune disease (e.g., lupus) and an infection (e.g., HIV infection) may be tested (n=10 or more; both patient cohorts are described) [165-167]. The suppression of misTTR-directed catabodies accompanied by enhanced polyreactive catabodies in ATTRwt contrasts with previous findings on patients with autoimmune disease, who display enhanced antigen-specific hydrolysis and suppressed polyreactive IgG class catabodies [52].

Importantly, accumulation of the naturally-occurring electrophilic antigens in disease can drive antibody class-switching to superantigens and amyloids in disease, an event that is otherwise prohibited in the immune response [167]. Accumulation of NE-misTTR derivatives in ATTRwt and ATTRm patient may result in synthesis of class-switched IgG and IgA antibodies. Proof for this hypothesis was provided by our studies on purified IgA class antibodies from ATTRwt patients shown in Table 4. misTTR hydrolysis by pooled purified IgA from these patients was readily detected (2.3 nmol hydrolysis/hour/µg IgA).

### B cell correlates of disease

Measurements of B cell binding to E-misTTR and E-hapten-1 serve as indices of misTTR selective (Fig 5A) and polyreactive (Fig 10D) catabody activities, respectively [37, 88]. Thus, the binding test may be used as an adjunct or an independent diagnostic test for ATTRwt and ATTRm. Purified CD19+ B cells expressing IgM class BCRs may be obtained from the blood of ATTRwt patients, ATTRm patients and control age-matched humans described in the preceding section by standard methods. The cells may be stained with E-misTTR, E-hapten-1 or control irrelevant antigens, followed by flow cytometric quantification of the number of stained B cells and the staining intensity. Reduced E-misTTR staining intensity of cells from the patient groups compared to control humans indicates the ability to diagnose ATTRT and ATTRm. Increased E-hapten-1 staining of cells from the patient groups may be compared to the control human group. Discrimination between the patient groups and controls may be determined from the assay sensitivity, specificity, precision, false positive rate and false negative rate parameters. Improvement in these parameters upon combining the E-misTTR staining data and E-hapten 1 staining data as in Table 5 indicates the ability to more accurately diagnose ATTRwt and ATTRm.

### Detection of misTTR as a disease marker

The blood of ATTRwt may contain soluble misTTR [65], and healthy humans may also be positive for small amounts of such misTTR species. Owing to their specificity for a neoepitope expressed by misTTR, the catabodies are unique reagents that may permit detection of the blood-borne misTTR species, thereby permitting diagnosis of ATTRwt. Such species are difficult to detect by conventional binding assays. The catabodies detect both soluble misTTR, which act as seeds for amyloid formation, and the TTR amyloid itself (see Example 1). The misTTR species may be detected, for example, by treatment of human blood or another human bodily fluid with a specific anti-misTTR catabody, followed by monitoring of the hydrolytic reaction using conventional antibodies that bind the fragments produced by digestion of the misTTR. Conventional antibodies that bind TTR fragments have been published [148]. The detection of the TTR fragments can be accomplished, for example, using enzyme-linked immunosorbent assays or electrophoresis followed by immunoblotting.

A well-characterized catabody from Example 1 may be employed for detection of misTTR in ATTRwt patients and control age-matched healthy humans described in Example 2. A finding of increased soluble misTTR species in ATTRwt patients using a specific misTTR catabody indicates the ability to use the catabody for ATTRwt diagnosis. The ability of the assay to discriminate between the patient and control groups may be determined from the assay sensitivity, specificity, precision, false positive rate and false negative rate.

### Abbreviations

AMC, aminomethylcoumarin; AAA-AMC, Ala-Ala-Ala-aminomethylcoumarin; Ag, antigen; AGE, advanced glycation end-product; ATTRm, hereditary TTR amyloidosis caused by mutant TTR; ATTRwt, wildtype TTR amyloidosis; AVU, arbitrary volume units; Aβ, amyloid β peptide; Aβ42, Aβ 1-42; Aβ40, Aβ 1-40; BCR, B cell receptor; C domain, constant domain; catabody, catalytic antibody; CDR, complementarity determining region; CHAPS, 3-[(3-cholamidopropyl) dimethylammonio]-1-propanesulfonate; CPM, count per min; E-Ag, electrophilic antigen; E-hapten, electrophilic hapten; E-misTTR, electrophilic misTTR; E-phyTTR, electrophilic phyTTR; EBV, Epstein-Barr virus; EAR-AMC, Glu-Ala-Arg-aminomethylcoumarin; EDTA, ethylenediaminetetraacetic acid; FITC, fluorescein isothiocyanate; FR, framework region; LPE, lipid peroxidation end-product; MMP9, matrix metalloproteinase-9; mIgM, monoclonal IgM; misTTR, misfolded TTR; NE-Aβ, naturally-occurring electrophilic Aβ; NE-misTTR, naturally-occurring electrophilic misTTR; Ova, ovalbumin; PBS, phosphate buffered saline; PECy7, phycoerythrin cyanin 7; phyTTR, physiological TTR; pIgM, polyclonal IgM; SAP, serum amyloid P component; SDS, sodium dodecyl sulfate;Tg, transgenic; Tg-TTR, transgenic mice expressing wildtype human TTR; Tg-V30M TTR, transgenic mice expressing mutant V30:M human TTR; ThT, Thioflavin-T;TTR, transthyretin; V domain, variable domain; V_{H} domain; heavy chain V domain; V_{L}, light chain V domain.

### References

1. Buxbaum, J.N., Trends Biochem Sci 2003,28:585-592.
2. Buxbaum et al., Cell Mol Life Sci 2009,66:3095-3101.
3. Sipe et al., Amyloid 2012,19:167-170.
4. Westermark et al., Amyloid 2003,10 Suppl 1:48-54.
5. Planque et al., J Biol Chem 2014,289:13243-13258.
6. Hammer et al., J Alzheimers Dis 2008,13:407-419.
7. Gertz et al., Biomed Pharmacother 1989,43:101-106.
8. Weiss et al., J Neurochem 2008,104:846-858.
9. Buchhave et al., Arch Gen Psychiatry 2012,69:98-106.
10. Williams et al., FEBS J 2011,278:3905-3917.
11. Sorgjerd et al., Biochem Biophys Res Commun 2008,377:1072-1078.
12. Bourgault et al., Biochem Biophys Res Commun 2011,410:707-713.
13. Zhao et al., Biochemistry 2013,52:1913-1926.
14. Pepys et al., Nature 2002,417:254-259.
15. Castro-Rodrigues et al., Acta Crystallogr D Biol Crystallogr 2011,67:1035-1044.
16. Pepys, M.B., Annu Rev Med 2006,57:223-241.
17. Sekijima et al., Hum Pathol 2011,42:1785-1791.
18. Westermark et al., Ups J Med Sci 2014.
19. Tanskanen et al., Ann Med 2008,40:232-239.
20. Jacobson et al., Hum Genet 1996,98:236-238.
21. Bulawa et al., Proc Natl Acad Sci U S A 2012,109:9629-9634.
22. Coelho et al., Neurology 2012,79:785-792.
23. Ackermann et al., Amyloid 2012,19 Suppl 1:43-44.
24. Coelho et al., N Engl J Med 2013,369:819-829.
25. Obici et al., Amyloid 2012,19 Suppl 1:34-36.
26. Kristen et al., Clin Res Cardiol 2012,101:805-813.
27. Janeway et al., Chapter 9: the humoral response. In Immunobiology: the immune system in health and disease. 6th edition. (Janeway, C.A. ed) pp. 367-408, Garland Publishing, New York, New York.
28. Zhu et al., Curr Biol 2012,22:872-880.
29. Gololobov et al., Mol Immunol 1999,36:1215-1222.
30. Gao et al., J Mol Biol 1995,253:658-664.
31. Sapparapu et al., J Biol Chem 2012,287:36096-36104.
32. Sun et al., J Immunol 1994,153:5121-5126.
33. Taguchi et al., J Biol Chem 2008,283:36724-36733.
34. Meixlsperger et al., Immunity 2007,26:323-333.
35. Kohler et al., Immunity 2008,29:912-921.
36. Kulathu et al., EMBO J 2008,27:1333-1344.
37. Planque et al., J Biol Chem 2004,279:14024-14032.
38. Nishiyama et al., J Mol Recognit 2006,19:423-431.
39. Paul et al., J Biol Chem 2003,278:20429-20435.
40. Paul et al., Adv Exp Med Biol 2012,750:56-75.
41. Oakley et al., J Neurosci 2006,26:10129-10140.
42. Davis et al., J Biol Chem 2004,279:20296-20306.
43. Miao et al., Am J Pathol 2005,167:505-515.
44. Veerhuis, R., Curr Alzheimer Res 2011,8:34-58.
45. Clausen et al., Eur J Neurosci 2011,34:110-123.
46. Kotzbauer et al., Arch Neurol 2012,69:1326-1331.
47. Wilcock et al., J Neuroinflammation 2004,1:24.
48. Salloway et al., N Engl J Med 2014,370:322-333.
49. Kou et al., J Alzheimers Dis 2011,27:23-38.
50. Paul et al., Preclinical catalytic immunotherapy of Alzheimer's disease. Keystone Symposium - Aging and Diseases of Aging. Tokyo, Oct 22-27 2012.
51. Nishiyama et al., Beneficial catalytic autoimmunity to amyloid beta peptide. Alzheimer's Association International Conference (AAIC12) Vancouver, British Columbia, July 14-19 2012.
52. Kalaga et al., J Immunol 1995,155:2695-2702.
53. Lacroix-Desmazes et al., Proc Natl Acad Sci U S A 2005,102:4109-4113.
54. Dooley et al., Dev Comp Immunol 2006,30:43-56.
55. Taguchi et al., J Biol Chem 2008,283:4714-4722.
56. Paul et al., J Biol Chem 2004,279:39611-39619.
57. Sharma et al., J Biol Chem 2009,284:33079-33087.
58. Hifumi et al., FASEB J 2012,26:1607-1615.
59. Le Minoux et al., Mol Immunol 2012,50:160-168.
60. Paul et al., Science 1989,244:1158-1162.
61. Shuster et al., Science 1992,256:665-667.
62. Takeuchi et al., Proteins 2007,66:716-725.
63. Quintas et al., J Biol Chem 2001,276:27207-27213.
64. Hurshman et al., Biochemistry 2004,43:7365-7381.
65. Kingsbury et al., Anal Biochem 2012,425:151-156.
66. Sueyoshi et al., Hum Pathol 2011,42:1259-1264.
67. Teixeira et al., J Biol Chem 2006,281:21998-22003.
68. Connors et al., Amyloid 2003,10:160-184.
69. Su et al., Amyloid 2012,19 Suppl 1:45-46.
70. Lindstrom et al., Immunotherapy 2014,6:141-153.
71. Lundberg et al., Febs J 2009,276:1999-2011.
72. Yang et al., Biochemistry 2013,52:2849-2861.
73. Xia et al., Biochemistry 2012,51:100-107.
74. Mitsuda et al., Mol Biotechnol 2007,36:113-122.
75. Beynon et al., Appendix III. Commercially available protease inhibitors. In: Proteolytic Enzymes: A Practical Approach (Second Edition). Eds. Beynon et al. Oxford: Oxford University Press; 2001:317-330.
76. Paul et al., J Biol Chem 2001,276:28314-28320.
77. Sousa et al., Faseb J 2005,19:124-126.
78. Mody et al., Int J Pept Protein Res 1994,44:441-447.
79. Reixach et al., Proc Natl Acad Sci U S A 2004,101:2817-2822.
80. Takeshita et al., Clin Exp Immunol 2001,125:340-344.
81. Chen et al., Am J Hematol 1993,44:276-279.
82. Foguel et al., Proc Natl Acad Sci U S A 2003,100:9831-9836.
83. Polosukhina et al., Immunol Lett 2006,103:75-81.
84. Baranova et al., Int Immunol 2010,22:671-680.
85. Schurr et al., Science 1966,152:1064-1066.
86. Cruys-Bagger et al., Febs J 2013,280:3952-3961.
87. Paul et al., J Biol Chem 1990,265:11910-11913.
88. Planque et al., J Biol Chem 2003,278:20436-20443.
89. Casali et al., Curr Top Microbiol Immunol 1996,210:167-179.
90. O'Nuallain et al., Proc Natl Acad Sci U S A 2002,99:1485-1490.
91. Kayed et al., Science 2003,300:486-489.
92. Graille et al., Proc Natl Acad Sci U S A 2000,97:5399-5404.
93. Neshat et al., Int Immunol 2000,12:305-312.
94. Goodyear et al., Semin Immunopathol 2005,26:463-484.
95. Nishiyama et al., J Biol Chem 2009,284:30627-30642.
96. Planque et al., J Immunol 2012,189:5367-5381.
97. Spencer et al., Immunology 1984,52:7-15.
98. Rossing, N., Lymphology 1978,11:138-142.
99. Pilati, C.F., Am J Physiol 1990,258:H748-753.
100. Gomes et al., Biochem J 2005,385:339-345.
101. Liu et al., J Mol Biol 2008,377:1236-1250.
102. Wang et al., Clin Immunol 1999,93:132-142.
103. Gibson et al., Aging Cell 2009,8:18-25.
104. Gavazzi et al., Lancet Infect Dis 2002,2:659-666.
105. Maas et al., J Immunol 1999,162:6526-6533.
106. Conley et al., Annu Rev Immunol 2009,27:199-227.
107. Liu et al., Nat Rev Neurol 2012,8:465-469.
108. Pollack et al., J. Am. Chem. Soc. 1989,111:5961-5962.
109. Ramsland et al., Biochem J 2006,395:473-481.
110. Janda et al., J Biol Chem 2012,287:35409-35417.
111. Haury et al., Eur J Immunol 1997,27:1557-1563.
112. Nissim et al., Historical Development of Monoclonal Antibody Therapeutics. In: Therapeutic Antibodies. Handbook of Experimental Pharmacology. Ed. Chernajovsky et al.: Springer Berlin Heidelberg; 2008:3-18.
113. Ponomarenko et al., Proc Natl Acad Sci U S A 2006,103:281-286.
114. Wootla et al., J Immunol 2008,180:7714-7720.
115. Wootla et al., Blood 2011,117:2257-2264.
116. Andrievskaya et al., Med Sci Monit 2000,6:460-470.
117. Saveliev et al., Immunol Lett 2003,86:291-297.
118. Gao et al., J Biol Chem 1994,269:32389-32393.
119. Uda et al., J Biosci Bioeng 2004,97:143-152.
120. Hifumi et al., J Biol Chem 2008,283:899-907.
121. Coutinho et al., Curr Opin Immunol 1995,7:812-818.
122. Bayry et al., Expert Rev Clin Immunol 2005,1:213-222.
123. Kaveri, S.V., Autoimmun Rev 2012,11:792-794.
124. Kohler et al., Immunol Today 1998,19:221-227.
125. Graille et al., J Biol Chem 2002,277:47500-47506.
126. Maftei et al., PLoS One 2012,7:e44516.
127. Lesne et al., Brain 2013,136:1383-1398.
128. Kalinin et al., Neurobiol Aging 2013,34:2361-2369.
129. Lane et al., Arthritis Rheum 2013,65:1116-1121.
130. Abdallah et al., Saudi J Kidney Dis Transpl 2013,24:950-958.
131. Calderon-Garciduenas et al., Biomed Res Int 2013,2013:161687.
132. Millucci et al., ScientificWorldJournal 2012,2012:293863.
133. Planque et al., AIDS Res Hum Retroviruses 2007,23:1541-1554.
134. Brown et al., J Biol Chem 2012,287:9940-9951.
135. Kreymann et al., Crit Care Med 2007,35:2677-2685.
136. Xu et al., J Immunol Methods 1992,146:241-247.
137. Taguchi et al., J Biol Chem 2008,283:4714-4722.
138. Sapparapu et al., J Biol Chem 2012,287:36096-36104.
139. Smith et al., Nat Protoc 2009,4:372-384.
140. Sapparapu et al., J Biol Chem 2009,284:24622-24633.
141. Tyutyulkova et al., Biochim Biophys Acta 1996,1316:217-223.
142. Liu et al., BMC Biotechnol 2007,7:78.
143. Nishiyama et al., Arch Biochem Biophys 2002,402:281-288.
144. Plante-Bordeneuve et al., Lancet Neurol 2011,10:1086-1097.
145. Dahlgren et al., J Biol Chem 2002,277:32046-32053.
146. Myatt et al., Proc Natl Acad Sci U S A 1994,91:3034-3038.
147. Takashima, A., Curr Alzheimer Res 2010,7:665-669.
148. Gustavsson et al., Am J Pathol 1994,144:1301-1311.
149. Bergstrom et al., Biochem Biophys Res Commun 2006,348:532-539.
150. Goldsteins et al., Proc Natl Acad Sci U S A 1999,96:3108-3113.
151. Reshetnyak et al., J Am Chem Soc 2007,129:16175-16182.
152. Paul et al., J Biol Chem 1992,267:13142-13145.
153. Ihse et al., Biochem Biophys Res Commun 2009,379:846-850.
154. Ihse et al., J Pathol 2008,216:253-261.
155. Teng et al., Lab Invest 2001,81:385-396.
156. Yi et al., Am J Pathol 1991,138:403-412.
157. Cardoso et al., FASEB J 2006,20:234-239.
158. Nissim et al., Historical development of monoclonal antibody therapeutics. In: Handb Exp Pharmacol. Edited by Chernajovsky Y, Nissim A. Verlag Berlin Heidelberg: Springer; 2008:3-18.
159. Gololobov et al., J Pharmacol Exp Ther 1998,285:753-758.
160. Vieira et al., Eur J Immunol 1988,18:313-316.
161. Goodnow et al., Nature 1988,334:676-682.
162. Smith et al., J Throm Haemos. 5 (Suppl. 2) O-M-063.
163. Berk et al., JAMA 2013,310:2658-2667.
164. Kyle et al., Am J Med 1996,101:395-400.
165. Paul et al., J Immunol 1997,159:1530-1536.
166. Planque et al., AIDS 2010,24:875-884.
167. Planque et al., Deficient synthesis of class-switched, HIV neutralizing antibodies to the CD4 binding site and correction by electrophilic gp120 immunogen AIDS 2014.

## Claims

1. An isolated catalytic antibody or fragment thereof immunologically specific for misfolded transthyretin for use in the treatment of transthyretin amyloidosis, wherein said misfolded transthyretin is transthyretin misfolded into soluble oligomers or particulate amyloid with β-structure, wherein said catalytic antibody catalyzes the hydrolysis of said misfolded transthyretin but not physiologically folded TTR, and wherein said catalytic antibody contains a nucleophilic site with peptide bond hydrolyzing activity that is inhibited by a serine protease inhibitor.

2. The catalytic antibody or fragment thereof for use of claim 1, wherein said catalytic antibody is an IgM.

3. The catalytic antibody or fragment thereof for use of claim 1, wherein said misfolded transthyretin is human.

4. The catalytic antibody or fragment thereof for use of claim 1, which is an IgM isolated from a healthy human without amyloidosis, an IgA isolated from a human with wildtype TTR amyloidosis, or a mouse immunized with the electrophilic analog of misfolded transthyretin, which contains electrophilic phosphonates and biotin substituents linked to Lys residues.

5. An *in vitro* method for detecting misfolded transthyretin in a test sample, said method comprising:
- contacting the test sample with a catalytic antibody or fragment thereof immunologically specific for misfolded transthyretin, wherein said catalytic antibody or fragment thereof catalyzes the hydrolysis of said misfolded transthyretin, wherein said misfolded transthyretin is transthyretin misfolded into soluble oligomers or particulate amyloid with β-structure, wherein said catalytic antibody does not catalyze the hydrolysis of physiologically folded TTR, and wherein said catalytic antibody contains a nucleophilic site with peptide bond hydrolyzing activity that is inhibited by a serine protease inhibitor, and,
- monitoring the hydrolysis of misfolded transthyretin.

6. An *in vitro* method for diagnosing transthyretin amyloidosis, said method comprising detecting the presence of misfolded transthyretin in a biological sample, wherein said detection comprises performing the method of claim 5.

7. An electrophilic analog of isolated misfolded transthyretin, wherein said electrophilic analog contains electrophilic phosphonates and biotin substituents linked to Lys residues of said misfolded transthyretin.

8. A method for isolating catalytic antibodies that hydrolyze misfolded transthyretin, said method comprising contacting a biological sample with the electrophilic analog of claim 7.

9. The electrophilic analog of claim 7, for use as a vaccine in the treatment or prevention of transthyretin amyloidosis.

10. The electrophilic analog of claim 7, suitable for use in the production of monoclonal catalytic antibodies in mice.

## Patentansprüche

1. Isolierter, katalytischer Antikörper oder Fragment davon, der/das immunologisch spezifisch für ein fehlgefaltetes Transthyretin ist, zur Verwendung bei der Behandlung einer Transthyretin-Amyloidose, wobei das genannte fehlgefaltete Transthyretin ein Transthyretin ist, das in lösliche Oligomere oder Partikelamyloid mit β-Struktur fehlgefaltet ist, wobei der genannte katalytische Antikörper die Hydrolyse des genannten fehlgefalteten Transthyretins, aber nicht von physiologisch gefaltetem Transthyretin katalysiert, und wobei der genannte katalytische Antikörper ein nukleophiles Zentrum mit einer Peptidbond-hydrolysierenden Aktivität enthält, die von einem Serinprotease-Inhibitor inhibiert wird.

2. Katalytischer Antikörper oder Fragment davon zur Verwendung gemäß Anspruch 1, wobei der genannte katalytische Antikörper ein IgM ist.

3. Katalytischer Antikörper oder Fragment davon zur Verwendung gemäß Anspruch 1, wobei das genannte fehlgefaltete Transthyretin menschlich ist.

4. Katalytischer Antikörper oder Fragment davon zur Verwendung gemäß Anspruch 1, der ein IgM, das von einem gesunden Menschen ohne Amyloidose isoliert ist, ein IgA is, das von einem Menschen mit Wildtyp TTR-Amyloidose oder von einer Maus isoliert ist, die mit dem elektrophilen Analog von fehlgefaltetem Transthyretin immunisiert ist, das elektrophile Phosphonate und mit Lys-Resten verbundene Biotin-Substituenten enthält.

5. *In vitro* -Verfahren zum Detektieren von fehlgefaltetem Transthyretin in einer Testprobe, wobei das genannte Verfahren umfasst:
- Kontaktieren der Testprobe mit einem katalytischen Antikörper oder Fragment davon, der/das immunologisch spezifisch für fehlgefaltetes Transthyretin ist, wobei der genannte katalytische Antikörper oder das Fragment davon die Hydrolyse des genannten fehlgefalteten Transthyretins katalysiert, wobei das genannte fehlgefaltete Transthyretin ein Transthyretin ist, das in lösliche Oligomere oder Partikelamyloid mit β-Struktur fehlgefaltet ist, wobei der genannte katalytische Antikörper die Hydrolyse des genannten physiologisch gefalteten TTR nicht katalysiert, und wobei der genannte katalytische Antikörper ein nukleophiles Zentrum mit einer Peptidbond-hydrolysierenden Aktivität enthält, die von einem Serinprotease-Inhibitor inhibiert wird, und
- Überwachen der Hydrolyse von fehlgefaltetem Transthyretin.

6. *In vitro* -Verfahren zum Diagnostizieren einer Transthyretin-Amyloidose, wobei das genannte Verfahren das Detektieren der Anwesenheit von fehlgefaltetem Transthyretin in einer biologischen Probe umfasst, wobei die genannte Detektion das Durchführen des Verfahrens gemäß Anspruch 5 umfasst.

7. Elektrophiles Analog von isoliertem, fehlgefaltetem Transthyretin, wobei das genannte elektrophile Analog elektrophile Phosphonate und Biotin-Substituenten umfasst, die mit Lys-Resten des genannten fehlgefalteten Transthyretins verbunden sind.

8. Verfahren zum Isolieren von katalytischen Antikörpern, die fehlgefaltetes Transthyretin hydrolysieren, wobei das genannte Verfahren das Kontaktieren einer biologischen Probe mit dem elektrophilen Analog gemäß Anspruch 7 umfasst.

9. Elektrophyles Analog gemäß Anspruch 7 zur Verwendung als ein Vakzin zur Behandlung oder Vorbeugung einer Transthyretin-Amyloidose.

10. Elektrophyles Analog gemäß Anspruch 7, das zur Verwendung bei der Produktion von monoklonalen katalytischen Antikörpern bei Mäusen geeignet ist.

## Revendications

1. Anticorps catalytique isolé ou fragment isolé de celui-ci, immunologiquement spécifique de la transthyrétine mal repliée, pour l'utilisation dans le traitement de l'amylose à transthyrétine, ladite transthyrétine mal repliée étant une transthyrétine mal repliée en oligomères solubles ou en amyloïde particulaire de structure β, ledit anticorps catalytique catalysant l'hydrolyse de ladite transthyrétine mal repliée mais pas de la TTR physiologiquement repliée, et ledit anticorps catalytique contenant un site nucléophile avec une activité d'hydrolyse de liaison peptidique qui est inhibée par un inhibiteur de sérine protéase.

2. Anticorps catalytique ou fragment de celui-ci, pour l'utilisation selon la revendication 1, ledit anticorps catalytique étant une IgM.

3. Anticorps catalytique ou fragment de celui-ci pour l'utilisation selon la revendication 1, dans lequel ladite transthyrétine mal repliée est humaine.

4. Anticorps catalytique ou fragment de celui-ci pour l'utilisation selon la revendication 1, qui est une IgM isolée à partir d'un humain sain sans amyloïdose, une IgA isolée à partir d'un humain avec une amyloïdose TTR de type sauvage, ou une souris immunisée avec l'analogue électrophile de transthyrétine mal repliée, qui contient des phosphonates électrophiles et des substituants de type biotine liés à des résidus Lys.

5. Procédé *in vitro* pour détecter la transthyrétine mal repliée dans un échantillon, ledit procédé comprenant :
- la mise en contact de l'échantillon d'essai avec un anticorps catalytique ou un fragment de celui-ci immunologiquement spécifique de la transthyrétine mal repliée, ledit anticorps catalytique ou fragment de celui-ci catalysant l'hydrolyse de ladite transthyrétine mal repliée, ladite transthyrétine mal repliée étant de la transthyrétine mal repliée en oligomères solubles ou en amyloïde particulaire de structure β, ledit anticorps catalytique ne catalysant pas l'hydrolyse de la TTR physiologiquement repliée, et ledit anticorps catalytique contenant un site nucléophile avec une activité d'hydrolyse de la liaison peptidique qui est inhibée par un inhibiteur de sérine protéase, et,
- le contrôle de l'hydrolyse de la transthyrétine mal repliée.

6. Procédé *in vitro* pour diagnostiquer une amyloïdose à transthyrétine, ledit procédé comprenant la détection de la présence de transthyrétine mal repliée dans un échantillon biologique, dans lequel ladite détection comprend la mise en œuvre du procédé de la revendication 5.

7. Analogue électrophile de transthyrétine mal repliée, isolée, ledit analogue électrophile contenant des phosphonates électrophiles et des substituants de type biotine liés à des résidus Lys de ladite transthyrétine mal repliée.

8. Procédé pour isoler des anticorps catalytiques qui hydrolysent la transthyrétine mal repliée, ledit procédé comprenant la mise en contact d'un échantillon biologique avec l'analogue électrophile de la revendication 7.

9. Analogue électrophile selon la revendication 7, pour l'utilisation comme vaccin dans le traitement ou la prévention de l'amyloïdose à transthyrétine.

10. Analogue électrophile selon la revendication 7, approprié pour l'utilisation dans la production d'anticorps catalytiques monoclonaux chez la souris.
